(19) Europäisches Patentamt
European Patent Office
Office européen des brevets

(11) **EP 3 209 194 B1**

(12) **EUROPÄISCHE PATENTSCHRIFT**

(45) Veröffentlichungstag und Bekanntmachung des Hinweises auf die Patenterteilung:
**07.12.2022   Patentblatt 2022/49**

(21) Anmeldenummer: **15787133.6**

(22) Anmeldetag: **20.10.2015**

(51) Internationale Patentklassifikation (IPC):
**A61B 3/12** *(2006.01)*     **A61B 3/00** *(2006.01)*
**A61B 5/00** *(2006.01)*     **H01L 51/50** *(2006.01)*
**H01L 33/00** *(2010.01)*

(52) Gemeinsame Patentklassifikation (CPC):
**A61B 3/12; A61B 3/0008; H01L 51/50**

(86) Internationale Anmeldenummer:
**PCT/EP2015/002076**

(87) Internationale Veröffentlichungsnummer:
**WO 2016/062398 (28.04.2016 Gazette 2016/17)**

(54) **VORRICHTUNG ZUR BELEUCHTUNG DES INTRAOKULAREN RAUMES**

DEVICE FOR ILLUMINATING THE INTRAOCULAR SPACE

PROCÉDÉ POUR ÉCLAIRER L'ESPACE INTRAOCULAIRE

(84) Benannte Vertragsstaaten:
**AL AT BE BG CH CY CZ DE DK EE ES FI FR GB GR HR HU IE IS IT LI LT LU LV MC MK MT NL NO PL PT RO RS SE SI SK SM TR**

(30) Priorität: **20.10.2014   DE 102014221263**
**22.12.2014   DE 102014226790**

(43) Veröffentlichungstag der Anmeldung:
**30.08.2017   Patentblatt 2017/35**

(73) Patentinhaber: **PHARMPUR GmbH**
**86343 Königsbrunn (DE)**

(72) Erfinder:
• **LINGENFELDER, Christian**
**89160 Dornstadt (DE)**
• **HESSLING, Martin**
**89075 Ulm (DE)**
• **KÖLBL, Phillip**
**89077 Ulm (DE)**

(74) Vertreter: **Hoefer & Partner Patentanwälte mbB**
**Pilgersheimer Straße 20**
**81543 München (DE)**

(56) Entgegenhaltungen:
**WO-A1-03/030791      JP-A- 2009 226 156**
**US-A1- 2004 004 846      US-A1- 2009 096 988**
**US-A1- 2009 146 583**

• **Strahlenschutzkommission: "Blendung durch natürliche und neue künstliche Lichtquellen und ihre Gefahren", , 15. Februar 2006 (2006-02-15), Seiten 1-24, XP055240641, Gefunden im Internet: URL:http://www.ssk.de/SharedDocs/Beratungs ergebnisse_PDF/2006/Blendung_Lichtquellen. pdf?__blob=publicationFile [gefunden am 2016-01-12]**

## Beschreibung

### Gebiet der Erfindung

Die Erfindung betrifft eine Vorrichtung zur Beleuchtung des intraokularen Raumes des menschlichen oder tierischen Auges. Die Vorrichtung kann zur transskleralen nicht invasiven Verwendung ausgestaltet sein, oder kann so ausgestaltet sein, dass der intraokulare Raum direkt durch Insertion einer Lichtquelle in den Augeninnenraum beleuchtet wird. In einer weiteren Ausführungsform ist die Vorrichtung so ausgestaltet, dass transsklerale und direkte Beleuchtung des intraokularen Raumes gleichzeitig stattfinden kann. Die Erfindung betrifft weiterhin die Verwendung der Vorrichtungen in diagnostischen und/oder chirurgischen Verfahren zur nicht-invasiven transskleralen Beleuchtung des intraokularen Raumes. Die Erfindung betrifft weiterhin ein Verfahren zur Anpassung der Intensität der transskleralen Beleuchtung des intraokularen Raumes des menschlichen oder tierischen Auges mittels Anpassung des Anpressdrucks zwischen der Kontaktfläche der Vorrichtung und der Sklera des menschlichen oder tierischen Auges.

### Hintergrund der Erfindung

Erkrankungen des Auges, wie etwa Grauer Star (Katarakt), Glaukom (Grüner Star), altersbedingte Makuladegeneration und Diabetes-bedingte Retinopathie, sowie Netzhautveränderungen bzw. Netzhautablösungen nehmen zu, bedingt unter anderem durch die höhere Lebenserwartung der Bevölkerung. Die Diagnose und Behandlung dieser Erkrankungen ist wichtig, hierfür ist die Beleuchtung des Augenhintergrunds (*Fundus oculi*) eine essentielle Technologie. Zum Beispiel ist die Beleuchtung des Augenhintergrunds nötig, um pathologische Veränderungen an der Retina lokalisieren und therapieren zu können. Auch in der Ophthalmochirurgie, dem Fachbereich, der sich mit der operativen Behandlung von organischen oder funktionellen Erkrankungen befasst, wären Behandlungen, die durch operative Maßnahmen im Intraokularraum stattfinden, ohne geeignete Beleuchtung des intraokularen Raums kaum möglich.

Die Beleuchtung des intraokularen Raumes (Endoillumination) kann durch eine interne Lichtquelle erfolgen, wobei diese Lichtquelle dann durch Insertion in den Augeninnenraum gebracht werden muss.

Zum Zwecke der internen Endoillumination wird üblicherweise eine Lichtquelle mit Hilfe eines Trokars am Pars-Plana-Bereich in den Intraokularraum insertiert, um die Netzhaut direkt zu beleuchten. Dieser Bereich wird gewählt, da dort kein spezialisiertes, funktionell wichtiges Gewebe vorhanden ist und daher der Schaden durch die Insertion überschaubar ist. Allerdings bedeutet jede Insertion einer Lichtquelle in den Augenraum einen Eingriff und kann eine Schädigung des Okulargewebes oder eine Entzündung oder Infektion verursachen. Generell sollte daher, wenn möglich, jede invasive Maßnahme vermieden werden, insbesondere bei einem diagnostischen Verfahren, bei dem die Endoillumination nur der Bewertung der Integrität der Augenbestandteile, zum Beispiel der Netzhaut dient.

Seit den frühen 1970er Jahren, als Robert Machemer als Erster eine geschlossene Vitrektomie durchführte, wurden Technologien und Verfahren zur intraokularen Endoillumination entwickelt. In vielfacher Hinsicht konnten Fortschritte erzielt werden, was sich z.B. durch enorme Miniaturisierung der chirurgischen Ausstattung und Instrumente zeigt. Neuerungen führten zu leistungsfähigeren Lichtquellen und neuen Endoilluminationssystemen, die allerdings immer noch Nachteile aufweisen, wie etwa die Notwendigkeit eines invasiven Verfahrens. Auch schädliche Einflüsse auf das Auge sind mit den bekannten Systemen immer noch vorhanden.

Abgesehen von den bereits diskutierten Nachteilen der internen Endoillumination, die durch die notwendige Insertion der Lichtquelle in das Augeninnere ausgelöst werden, besteht auch noch ein weiteres Potential dieser Technik, das Okulargewebe des Patienten zu schädigen, da die Endoilluminatoren bei vitreoretinalen Eingriffen durch die direkte Beleuchtung der Netzhaut alle natürlichen Schutzmechanismen des Auges umgehen. Deshalb kann die Beleuchtung durch phototoxische oder thermische Wechselwirkungen mit dem Gewebe das Gewebe schädigen. Das gilt insbesondere für das sehr empfindliche Gewebe der Netzhaut.

Um dies zu vermeiden, müssen die Endoilluminationssysteme hohen Sicherheitskriterien genügen. Die eingesetzten Lichtquellen müssen derart ausgelegt sein, dass ihre abgegebene Strahlung keine toxischen Effekte auf der Retina hervorruft. Andererseits müssen die Lichtquellen aber auch derart ausgelegt sein, dass ihre abgegebene Strahlung für eine optimale Endoillumination sorgt. Das bedeutet, dass die durch die Lichtquelle erzeugte Helligkeit im intraokularen Raum eine Beurteilung des beleuchteten Gewebes erlaubt, so wie für gute Sichtverhältnisse für den Chirurg während des Eingriffes sorgt. Die Empfehlung der Strahlenschutzkommission mit dem Titel "Blendung durch natürliche und neue künstliche Lichtquellen und ihre Gefahren" vom 15.02.2016, Seiten 1 bis 24, abzurufen unter https://www.ssk.de/Shared-Docs/Beratungsergebnisse PDF/2006/Blendung Lichtque llen.pdf? blob=publicationFile gibt Anleitungen und Empfehlungen, wie Lichtquellen ausgestalten sein sollen, um Schäden vom Auge abzuwenden. Die Empfehlungen betreffen hauptsächlich Situationen wie Sonnenlicht und KFZ-Scheinwerfer im Straßenverkehr, und Umgang mit Laserquellen im Arbeitsumfeld. Lichtquellen zur intraokularen Beleuchtung werden nicht diskutiert.

Üblicherweise eingesetzte vitreoretinale Endoilluminatoren für Vitrektomien in der Ophthalmochirurgie verwenden Lichtleiter, in die das Licht eingekoppelt wird. Diese Vorrichtungen werden an der Pars plana, nahe der Hornhaut, in den intraokularen Raum insertiert, um den intraokularen Raum auszuleuchten. Ausführungsformen des Stands der Technik, die nach der Insertion am Auge verbleiben, und somit ein Halten von außen unnötig machen, werden als Chandelier-Illuminatoren bezeichnet. Die Vorrichtungen verwenden üblicherweise eine Xenon-, Quecksilber-, oder Halogenlichtquelle. Xenonlichtquellen haben den Nachteil, dass ihr Spektrum üblicherweise hohe Blau- und UV-Anteile beinhaltet, die auf die Retina toxisch wirken. Bei Halogenlichtquellen wird dieser Nachteil vermieden. Halogenlichtquellen erzeugen jedoch ein gelbliches Licht, das die Wahrnehmung verfälschen kann, und somit die Sichtverhältnisse im beleuchteten intraokularen Raum suboptimal werden lässt. Es besteht somit also ein Bedarf an neuartigen Lichtquellen, die ein Spektrum haben, das weder toxisch wirkt, noch die Wahrnehmung durch eine Farbgebung unkontrolliert verfälscht.

Neuere Alternativen nutzen eine in das Operationsgerät integrierte Leuchtdiode (LED), die über lange Lichtfasern das Licht in das Augeninnere abgibt. Es sind auch handgehaltene Geräte bekannt, die ebenfalls das Licht über eine Lichtfaser ins Auge einkoppeln. Die US-Patentanmeldungsveröffentlichung US 2004/0004846 A1 offenbart ein solches handgehaltenes Gerät, das im Inneren des Gehäuses eine LED aufweist, deren emittiertes Licht über einen Lichtleiter zu dessen distalen Ende geleitet wird. Dieses distale Ende wird in den Augeninnenraum eingeführt, um den intraokularen Raum zu beleuchten. Das Gerät muss vom Anwender mit der Hand geführt und gehalten werden, und weist keine Fixiervorrichtung auf, die in der Lage ist, das Gehäuse des Geräts an der Sklera des zu beleuchtenden Auges zu fixieren. Von Nachteil ist bei diesen aus dem Stand der Technik bekannten Systemen, dass durch das Einkoppeln der von der Lichtquelle abgegebenen Strahlung in den Lichtleiter und den Transport zum Wirkungsort, nämlich dem intraokularen Raum, hohe Leistungsverluste auftreten, die durch die Verwendung einer stärkeren Lichtquelle kompensiert werden müssen. Diese stärkeren intensiveren Lichtquellen sind zudem sehr teuer. Eine andere Möglichkeit besteht darin, durch eine präzise Ankopplung der Lichtquelle an die lichtleitenden Fasern der Lichtleiter den Leistungsverlust zu reduzieren, was jedoch aufwendig ist. Zudem besteht bei der Verwendung von Lichtleitern die Gefahr, dass das insertierte Lichtleiterende zumindest kurzzeitig näher als erwünscht an die Netzhaut heranreicht, wodurch die Netzhaut in kürzester Zeit photochemisch und/oder thermisch geschädigt werden kann.

Alternativ zu internen Endoilluminationssystemen, die der Insertion der Lichtquelle in das Augeninnere bedürfen, werden auch schon seit langem einfache Lampen verwendet, mit denen der Augenarzt das Auge beleuchtet. Diese Lampen erfordern jedoch das Halten oder Führen durch die Hand des Arztes. Stationäre Lampen wie etwa die Spaltlampe finden schon seit über hundert Jahren in der Okulardiagnostik Verwendung.

Weiterhin wird im Stand der Technik (DE 10 2010 016 629 A1) eine Vorrichtung vorgeschlagen, bei der auf die Insertion verzichtet wird. Die von einer Lichtquelle abgegebene Strahlung wird hier über eine Linse, die auf die Hornhaut des Auges aufzulegen ist, durch die Pupille in den Augenhintergrund geleitet. Die Lichtquelle ist hierbei in der Linse angeordnet. Von Nachteil ist hier, dass die von der Lichtquelle abgegebene Strahlung durch Okulargewebe hindurch strahlen muss, das einen hohen Brechungsindex hat, wie etwa die Linse. Dies hat zur Folge, dass nur ein geringer Anteil der von der Lichtquelle abgegebenen Strahlung am Wirkungsort, nämlich der Retina, ankommt.

Wie oben beschrieben, nehmen Pathologien des Auges und somit der Bedarf für Diagnose- oder Behandlungsverfahren dieser Pathologien zu. Ein sehr wichtiges Werkzeug ist eine zur Ausleuchtung des Augenhintergrunds geeignete Vorrichtung. Diese sollte einerseits nicht schädlich für das Auge bzw. das Okulargewebe sein, den Augenhintergrund gut ausleuchten, ein zur Sichtbarmachung der Membranen geeignetes Licht abgeben, und gleichzeitig preiswert, einfach herzustellen und einfach zu verwenden sein.

Es war daher Aufgabe der Erfindung, eine verbesserte, effizientere Vorrichtung bereitzustellen, die das Okulargewebe nicht oder nicht nachhaltig schädigt, die eine Ausleuchtung des gesamten intraokularen Raumes ermöglicht, ohne das Okulargewebe, zum Beispiel durch photochemische oder thermische Interaktion mit der von der Vorrichtung abgegebenen Strahlung, zu schädigen.

Außerdem besteht, insbesondere in der Diagnostik, ein Bedarf für eine Vorrichtung, die eine Endoillumination ohne Insertion der Lichtquelle in den Augeninnenraum und somit ohne Eingriff und Schädigung von Okulargewebe ermöglicht.

Weiterhin war es eine Aufgabe der Erfindung, eine verbesserte, effizientere Vorrichtung bereitzustellen, die eine gleichmäßige Ausleuchtung des gesamten intraokularen Raumes ermöglicht, ohne das Okulargewebe, zum Beispiel durch photochemische oder thermische Interaktion mit der von der in der Vorrichtung enthaltenen Lichtquelle abgegebenen Strahlung, zu schädigen.

Eine weitere Aufgabe der Erfindung bestand darin, eine Vorrichtung bereitzustellen, die sowohl für eine transsklerale, nicht invasive als auch für eine direkte Beleuchtung des Intraokularraumes des Auges eingesetzt werden kann, die somit einerseits ohne Verletzung von Augengewebe fixiert werden kann und trotzdem eine gute Ausleuchtung zulässt, die ggf. aber auch mit einer intraokularen Lichtquelle, die in den Augeninnenraum einbracht wird, versehen werden kann, wenn eine optimale Ausleuchtung des Augenhintergrundes, z.B. in einem chirurgischen Eingriff notwendig ist.

Diese Aufgaben werden gelöst durch die Vorrichtung wie sie in den Ansprüchen, insbesondere in Anspruch 1 und 2 definiert ist. Insbesondere wird eine Vorrichtung zur gleichmäßigen Ausleuchtung des gesamten intraokularen Raumes zur Verfügung gestellt, die eine Lichtquelle aufweist, die für die Verwendung in der Ophthalmologie geeignet ist und die Bedingungen der DIN EN ISO 15004-2:2014 erfüllt. Die Lichtquelle kann eine LED oder eine OLED und somit ein Flächenstrahler sein. Durch die Verwendung eines Flächenstrahlers wird die Strahlung verteilt und die maximal zu erwartenden Punktintensitäten werden enorm reduziert.

Die Vorrichtung weist weiterhin ein Gehäuse mit einer Oberfläche auf, die während der Verwendung in Kontakt mit der Augenoberfläche bzw. einem transkonjunktivalen Bereich steht.

Ein Vorteil der erfindungsgemäßen Vorrichtung ist es, dass sie einfach zu bedienen ist und während des Eingriffs nicht mit der Hand gehalten oder geführt werden muss. Dies wird einerseits erreicht, indem die Vorrichtung fixierbar ist. Die Vorrichtung kann so ausgebildet sein, dass sie mit einer Vorrichtung, die zur Fixierung am Auge geeignet ist, üblicherweise einer als Lidsperrer verwendeten Einrichtung, in solcher Art und Weise kombiniert werden kann, dass sie stabil in Kontakt mit der Sklera des auszuleuchtenden Auges steht. Hierbei ist die Vorrichtung von außen an der Sklera fixierbar, indem sie an die Oberfläche der Sklera angepresst wird. Die Vorrichtung kann auch zur Fixierung allein oder zusätzlich zur transkonjuktivalen Insertion geeignet sein.

Die Vorrichtung kann zusätzlich oder alternativ eine Lichtsonde wie in den Ansprüchen definiert aufweisen, deren geometrische Form die Vorrichtung während der Verwendung nach Insertion der Lichtsonde in den intraokularen Raum wie bei einem Chandelier-Illuminator üblich, fixiert, z.B. an der Sklera. Die geometrische Form der Lichtsonde kann zum Beispiel konisch sein, wobei sich der Außendurchmesser der Lichtsonde in distaler Richtung vom LED-Gehäuse weg erhöht. Die Lichtsonde kann auch derartig gestaltet sein, dass sie im Wesentlichen zylindrisch ist, und eine Ausbuchtung am distalen Ende aufweist. Die Ausbuchtung kann konisch, doppelkegelstumpfförmig, oder ballig sein. Die Unteransprüche enthalten vorteilhafte Weiterbildungen.

Die Aufgabe wurde weiterhin dadurch gelöst, dass die Vorrichtung der vorliegenden Erfindung aus Materialien hergestellt werden kann, die leicht zu sterilisieren sind, was auch eine leicht zu sterilisierende Gesamtvorrichtung zur Verfügung stellt.

Außerdem wird die Vorrichtung mit einer miniaturisierten Elektronik betrieben, die die Lichtquelle mit Spannung versorgt, zum Beispiel durch eine Knopfzelle oder einen Kondensator. Diese Vorrichtung ist eine preiswerte Alternative zu Vorrichtungen des Stands der Technik, und kann als Produkt zur einmaligen Verwendung vertrieben und angewendet werden.

Die verbesserte Vorrichtung der vorliegenden Erfindung ermöglicht auch ein Verfahren zur Anpassung der Intensität der Ausleuchtung, ohne zum Beispiel die Leistung der Lichtquelle zu verändern. Dies wird dadurch zur Verfügung gestellt, dass die Vorrichtung derartig an der Sklera vorübergehend anbringbar ist, zum Beispiel durch Anbringung der Vorrichtung mittels einer Fixiervorrichtung an einem Lidsperrer, dass ihre Position in Richtung zu der oder weg von der mit ihr in Kontakt stehenden Sklera des auszuleuchtenden Auges, verändert werden kann. Dabei erhöht eine Bewegung zu der Sklera hin den Anpressdruck, wodurch die Dicke der Sklera, und damit die Streuung der Strahlung in der Sklera, verringert wird. Dadurch wird die Transmission der Strahlung, und damit die Intensität der Ausleuchtung, gesteigert. Zudem kann auch durch die Auswahl des Bereiches des Auges, an dem die Vorrichtung anzubringen ist, die Intensität der Ausleuchtung variiert werden, da die Beleuchtungsintensität im intraokularen Raum von dem Abstand der Kontaktstelle zwischen Vorrichtung und Augenoberfläche zu dem zu beleuchtenden intraokularen Raum, wie etwa der Netzhaut, abhängt. Wird die Vorrichtung also zum Beispiel am Pars plana Bereich angebracht, so wird die Ausleuchtung weniger intensiv sein, als wenn ein Bereich des Auges ausgewählt wird, der lediglich aus Sklera und Aderhaut besteht, wie etwa der Augenrückraum.

## Beschreibung der Figuren

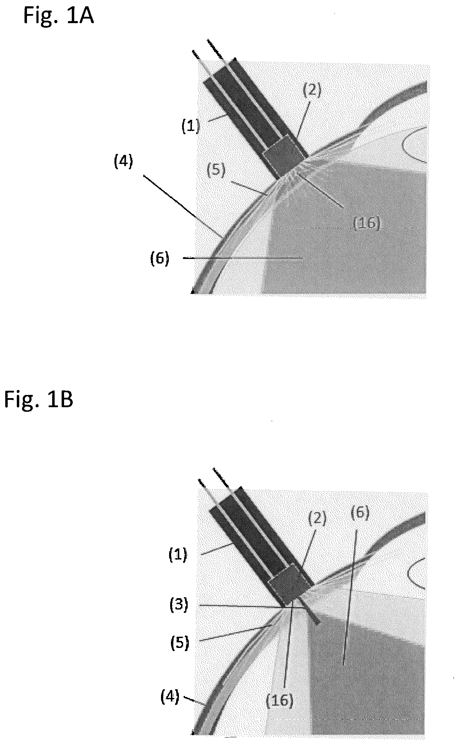

Figur 1: Prinzipskizzen der transskleralen Endoillumination in zwei Ausführungsformen Die Vorrichtungen weisen ein LED-Gehäuse (1), eine LED (2), und gegebenenfalls eine Lichtsonde (3) auf. Die von der LED abgegeben Strahlung wird im Sklera-Gewebe (4) gestreut (5) und leuchtet den Intraokularraum (6) aus. A zeigt die externe Endoillumination, bei der die Vorrichtung von außen an die Sklera aufgelegt wird, und eine Durchstoßung der Sklera nicht stattfindet. B zeigt die Kombination aus der externen Endoillumination der Ausführungsform aus Figur 1A mit einer internen Endoillumination, die durch eine insertierte Lichtsonde zur Verfügung gestellt wird.

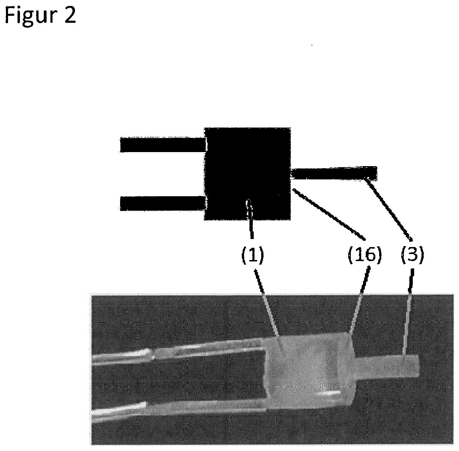

Figur 2 zeigt ein modifiziertes LED-Gehäuse (1) mit konischer Lichtsonde (3). Der Durchmesser der Lichtsonde nimmt zum LED-Gehäuse hin ab. Für diese Ausführungsform wurde das Epoxidharz-Gehäuse der LEDs so abgedreht, dass eine kleine Lichtsonde entsteht. Die so entstandene Lichtsonde kann am Pars plana durch die Sklera insertiert werden und den intraokularen Raum beleuchten. Nach Insertion steht die Vorrichtung über die Kontaktfläche (16) des LED-Gehäuses mit der äußeren Oberfläche der Sklera in Verbindung und verbleibt wie ein Chandelier-Illuminator am Ort der Insertion.

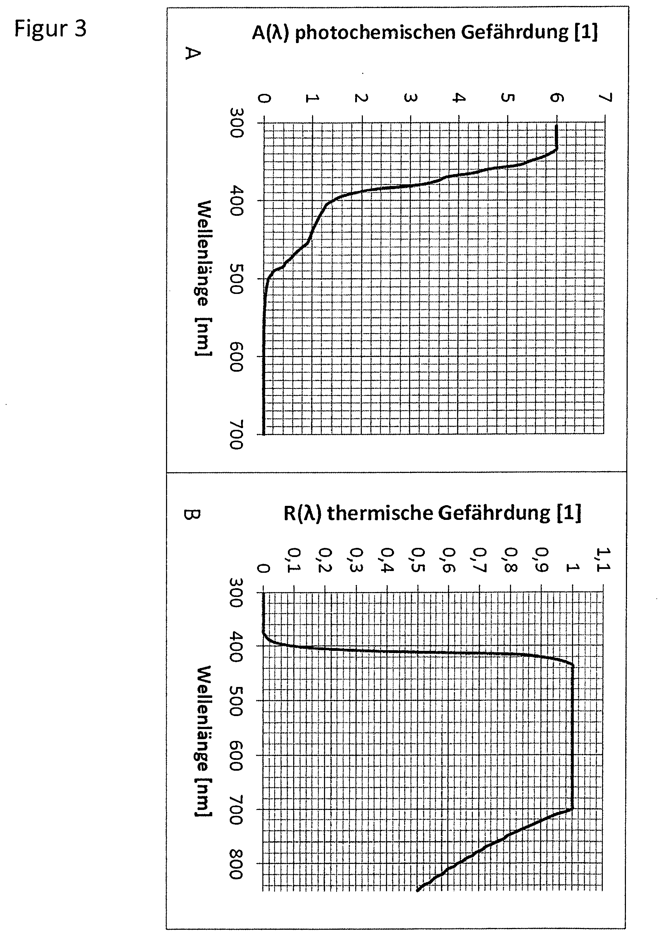

Figur 3 zeigt die Bewertungsfunktion für die photochemische Gefährdung $A(\lambda)$ in Teil A und die Bewertungsfunktion für die thermische Gefährdung $R(\lambda)$ in Teil B.

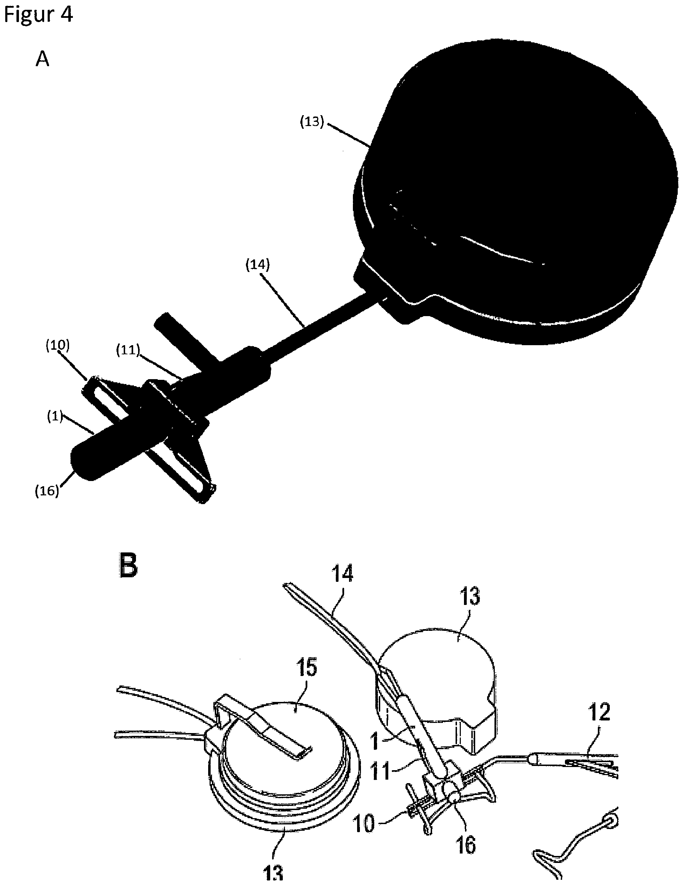

Figur 4 zeigt eine Ausführungsform zur nicht-invasiven gleichmäßigen transskleralen Beleuchtung des intraokularen Raumes. Das LED-Gehäuse (1) weist an einer äußeren Oberfläche einen Gewindegang (11) auf. Das Gehäuse ist über eine elektrische Leitung (14) mit einer Spannungsquelle in einem Gehäuse (13) verbunden. Das LED-Gehäuse ist über den Gewindegang (11) mit einem Aufsatz (10) verbunden.

A: Schematische Darstellung; B: photographische Darstellung, die die Ausführungsform zur nicht-invasiven gleichmäßigen transskleralen Beleuchtung des intraokularen Raumes zeigt, die über den Aufsatz (10) mit einem Lidsperrer (12) verbunden ist

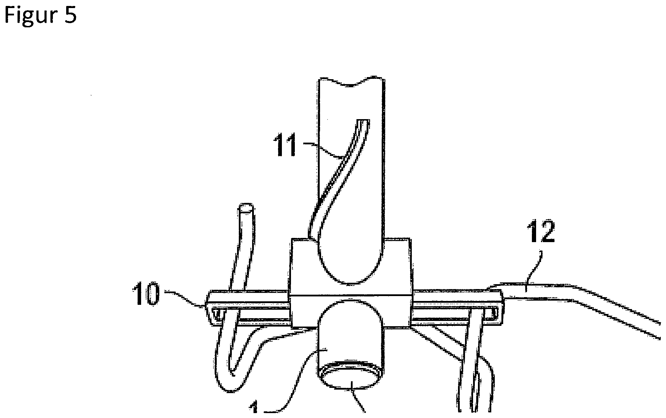

Figur 5 zeigt in vergrößerter Form die Ausführungsform zur nicht-invasiven gleichmäßigen transskleralen Beleuchtung des intraokularen Raumes aus Figur 4B, die über den Aufsatz (10) mit einem Lidsperrer (12) verbunden ist.

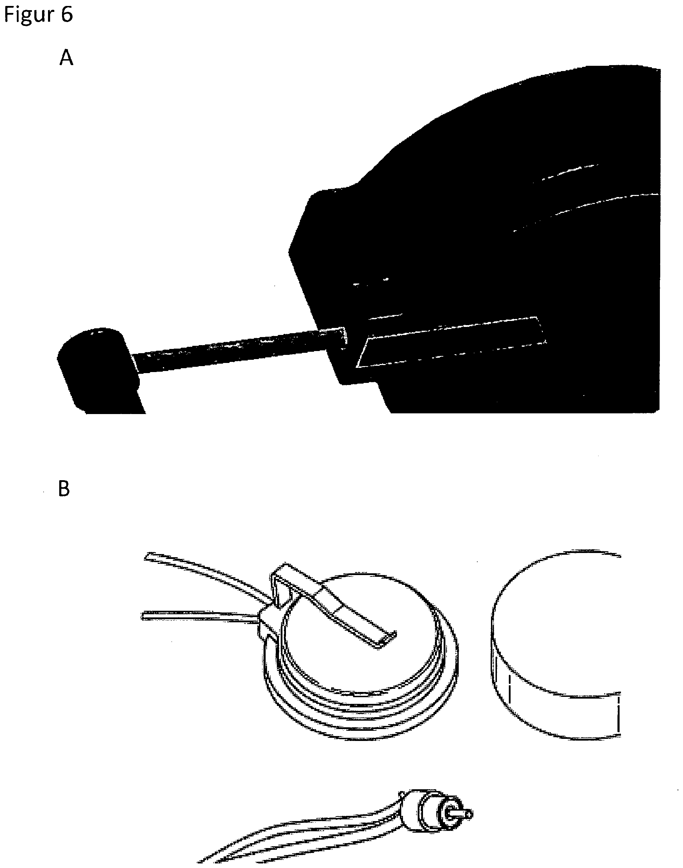

Figur 6 zeigt eine Ausführungsform der vorliegenden Erfindung, bei der die Fixiervorrichtung in Form einer Lichtsonde (3) ausgestaltet ist. A: Schematische Darstellung; B: photographische Darstellung

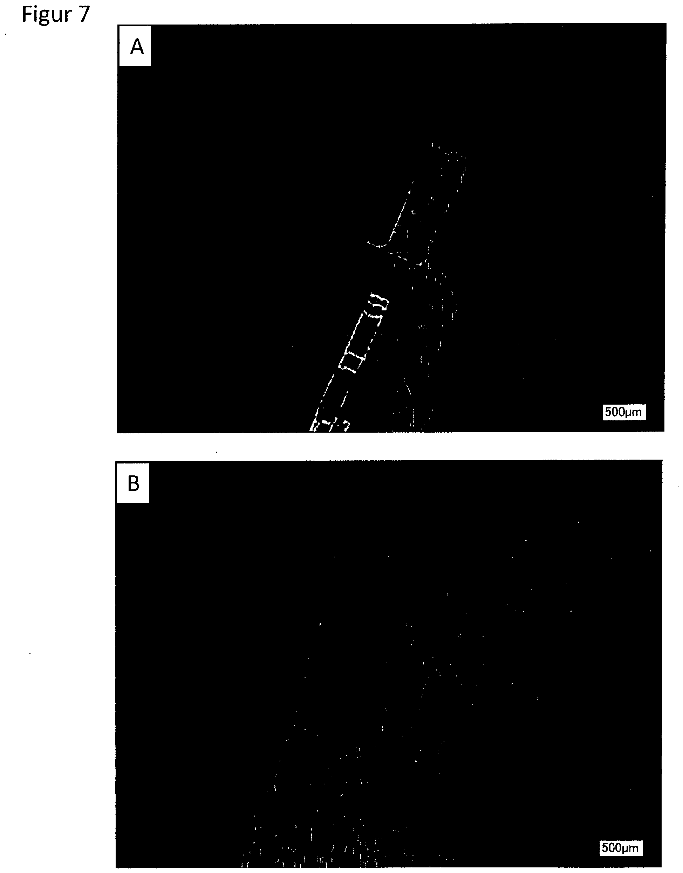

Figur 7A zeigt eine weitere vorteilhafte Ausführungsform eines modifizierten LED-Gehäuses mit einer Lichtsonde. Die geometrische Form der Lichtsonde ist in Figur 7B im Detail gezeigt. Die Form der Lichtsonde ermöglicht die Verankerung der LED in der Sklera des Auges wie für die in Figur 2 gezeigte Ausführungsform. Die Lichtsonde ist im Wesentlichen zylindrisch geformt, wobei die Lichtsonde einen im Bezug auf das LED-Gehäuse proximalen und einen distalen Bereich aufweist, wobei der Durchmesser im proximalen Bereich im Wesentlichen konstant bleibt, und wobei sich der Durchmesser im distalen Bereich zunächst auf einen maximalen Außendurchmesser erhöht, und dann am distalen Ende der Lichtsonde vom Gehäuse in Richtung von dem Gehäuse weg wieder auf den Durchmesser des proximalen Bereiches verjüngt. Der maximale Außendurchmesser der Lichtsonde ist 20 Gauge.

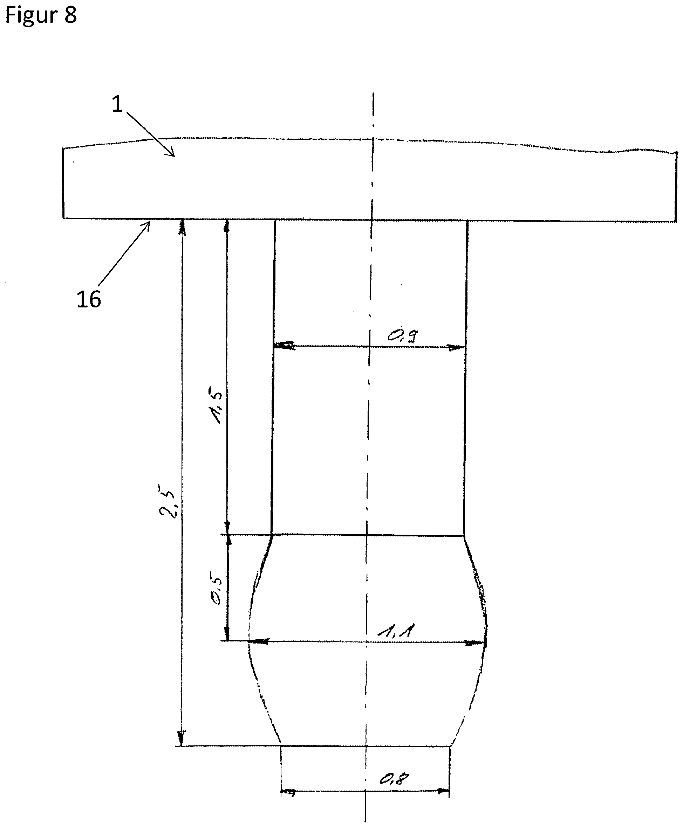

Figur 8 zeigt eine detaillierte Zeichnung der in Figur 7 gezeigten Lichtsonde.

## Definitionen

Unter Lichtquelle wird hierin eine LED oder eine OLED verstanden. Wenn eine Ausführungsform als beispielhaft eine LED aufweisend beschrieben wird, so ist dies so zu verstehen, dass die Ausführungsform auch eine OLED anstatt der LED aufweisen kann.

Die erfindungsgemäße Vorrichtung kann in einer Ausführungsform mittels einer von außen am Auge eingesetzten Vorrichtung am Auge fixierbar sein. Ein Beispiel für eine solche Vorrichtung ist ein Lidsperrer. Die Verwendung des Begriffes "Lidsperrer" ist lediglich als beispielhaft für derartige Vorrichtung zu verstehen, und nicht auf diesen begrenzt. Wenn daher in der folgenden Beschreibung der Begriff "Lidsperrer" verwendet wird, so wird hierunter jede Art von am Auge fixierbarer Vorrichtung verstanden, es sei denn, der Kontext spricht dagegen.

## Detaillierte Beschreibung der Erfindung

Die vorliegende Erfindung stellt eine Vorrichtung zur Ausleuchtung des gesamten intraokularen Raumes eines menschlichen oder tierischen Auges zur Verfügung, die eine Lichtquelle, wie etwa eine Leuchtdiode (LED) oder eine organische Leuchtdiode (OLED); ein Gehäuse, das die LED umgibt, und das gegebenenfalls einen Diffusor aufweist, der eine zur Auflegung an die Sklera eines menschlichen oder tierischen Auges geeignete Kontaktfläche aufweist und/oder zur transkonjunktivalen Insertion geeignet ist, und der die in Richtung der Kontaktfläche hin emittierte Strahlung unregelmäßig streut; eine mit der Lichtquelle verbundene Spannungsquelle; und eine Fixiervorrichtung zum Fixieren des Gehäuses wie in den Ansprüchen 1 und/oder 2 definiert aufweist; wobei die Lichtquelle eine thermische Belastung radiometrisch unter Berücksichtigung der maximalen Intensität der Abstrahlcharakteristik gemessen von weniger als 0,7 W/cm$^2$ erzeugt, und wobei die Lichtquelle den Augeninnenraum um nicht mehr als 10 °C erwärmt, und wobei das Spektrum der Lichtquelle keine Strahlung von weniger als 400 nm Wellenlänge aufweist. Die vorliegende Erfindung stellt eine Vorrichtung zur Ausleuchtung des gesamten intraokularen Raumes eines menschlichen oder tierischen Auges zur Verfügung, die eine Lichtquelle, wie etwa eine Leuchtdiode (LED) oder eine organische Leuchtdiode (OLED); ein Gehäuse, das die LED umgibt, und das gegebenenfalls einen Diffusor aufweist, der eine zur Auflegung an die Sklera eines menschlichen oder tierischen Auges geeignete Kontaktfläche aufweist, und der die in Richtung der Kontaktfläche hin emittierte Strahlung unregelmäßig streut; eine mit der Lichtquelle verbundene Spannungsquelle; und eine Fixiervorrichtung zum Fixieren des Gehäuses an der Sklera wie in den Ansprüchen 1 und/oder 2 definiert aufweist; wobei die Lichtquelle eine thermische Belastung radiometrisch unter Berücksichtigung der maximalen Intensität der Abstrahlcharakteristik gemessen von weniger als 0,7 W/cm$^2$ erzeugt, und wobei die Lichtquelle den Augeninnenraum um nicht mehr als 10 °C erwärmt, und wobei das Spektrum der Lichtquelle keine Strahlung von weniger als 400 nm Wellenlänge aufweist. Dadurch wird die Phototoxizität der durch die Lichtquelle abgegebenen Strahlung auf der Retina des Auges gegenüber Lichtquellen des Stands der Technik, die Strahlung mit einer Wellenlänger von weniger als 400 nm aufweisen, gesenkt, wodurch die maximale Expositionszeit $t_{max}$ in einem Abstand von sowohl 18 mm als auch 1 mm zwischen Kontaktfläche des Gehäuses der Lichtquelle und der Retina des menschlichen oder tierischen Auges gesteigert wird. Die maximale Expositionszeit $t_{max}$ ist größer, wenn die Phototoxizität der durch die Lichtquelle abgegebenen Strahlung kleiner ist. Zur Bestimmung der maximalen Expositionszeit ist ein Grenzwert von 10 J/cm$^2$ festgelegt. Dieser dient dazu ein maximales Maß an emittierter Strahlung festzulegen, die die Netzhaut ohne Schäden übersteht. Die maximale Expositionszeit $t_{max}$ soll so groß sein, dass eine Durchführung des mit der Beleuchtung einhergehenden diagnostischen oder chirurgischen Verfahrens möglich ist. Bevorzugt ist eine maximale Expositionszeit von mindestens 10 Minuten, oder von mindestens 60 Minuten.

Das Gehäuse umgibt die Lichtquelle und weist einen proximalen und distalen Bereich im Bezug auf die mit der Lichtquelle verbundene Spannungsquelle auf. Das Gehäuse kann transparent sein. Zumindest der distale Bereich kann aber auch aus einem Material hergestellt sein, das als Diffusor wirkt, und somit das von der Lichtquelle abgegebene Licht unregelmäßig streut. Die Eigenschaften des Diffusors werden durch das Material des Diffusorbereichs des Gehäuses und dessen Brechungsindex n bestimmt. Der Brechungsindex von trockener Luft bei Normatmosphäre beträgt etwa n = 1. Um beim Übergang der Strahlung vom Diffusorbereich des Gehäuses durch die Sklera und den Glaskörper in den intraokularen Raum hinein die Strahlung zu streuen, was für eine gleichmäßige Beleuchtung des intraokularen Raumes sorgt, muss der Brechungsindex des Diffusorbereichs des Gehäuses größer sein als der Brechungsindex in dem zuletzt durchstrahlten Material. Dies ist der Glaskörper, der zwischen Sklera und der zu beleuchtenden Netzhaut liegt. Der Brechungsindex des Glaskörpers ist etwa n = 1,3367. Folglich sollte der Brechungsindex des Diffusorbereichs des Gehäuses größer sein als n = 1,3367, um eine Streuung und damit eine gleichmäßige Beleuchtung der Netzhaut zu ermöglichen. Die Brechungsindizes des Diffusorbereichs des Gehäuses und des Glaskörpers werden hierin in Bezug auf den Brechungsindex der Luft wie oben beschrieben bezogen. In einer Ausführungsform hat das Material, aus dem der Diffusorbereich des Gehäuses hergestellt ist, einen Brechungsindex von mindestens n = 1,5. Ein Gehäuse, das aus einem Material hergestellt ist, das einen höheren Brechungsindex aufweist, ist bevorzugt, weil dadurch die Winkelbereiche für die Totalreflexion innerhalb des Gehäuses und der Lichtsonde vergrößert werden und damit Verluste durch den seitlichen Austritt, der zum Beispiel Blendung des Chirurgen hervorrufen kann, reduziert werden.

Zudem weist der Diffusorbereich des Gehäuses eine äußere Oberfläche auf, die so ausgestaltet sein kann, dass sie an der Oberfläche der Sklera so anbringbar ist, dass ein bündiger Kontakt entsteht. Diese Oberfläche wird hierin als Kontaktfläche bezeichnet. Das Gehäuse mit Lichtquelle kann auch eine Trokar-förmige Vorrichtung sein, die eine LED oder OLED enthält und transkonjunktival insertierbar ist, wobei das Gehäuse in dieser Ausführungsform ebenfalls einen Diffusorbereich aufweisen kann. Eine geeignete Vorrichtung ist in den Figuren gezeigt und wird unten beschrieben.

Die Lichtquelle kann eine LED sein und einem Außendurchmesser von nicht mehr als 3 mm haben, da LED dieser Größe noch nicht zu klein sind, um sie bearbeiten zu können. LED mit kleineren Ausmaßen, zum Beispiel mit einem quadratischen Grundriss von 0,2 mm, können ohne weitere Bearbeitung komplett in den Augeninnenraum eingebracht werden. In dieser Ausführungsform wird die Beleuchtungsvorrichtung der vorliegenden Erfindung, zum Beispiel über ein Trokar, in den intraokularen Raum gebracht. Der Sitz der Lichterzeugung ist demnach im intraokularen Raum, was den Wirkungsgrad zwischen eingesetzter Energie zur Lichterzeugung und erzielter Beleuchtung im intraokularen Raum signifikant steigert, da das erzeugte Licht kein okulares Gewebe zu durchdringen hat, bevor es am Einsatzort ankommt.

Das Gehäuse der LED kann flach, konkav oder konvex sein. Die Form des Gehäuses hat Einfluss auf die Lichtverteilung außerhalb der LED aber insbesondere auf den Druck auf die Sklera und damit die Transmissionseigenschaften der Sklera.

Weiterhin sollte die Vorrichtung gemäß der DIN EN ISO 15004-2:2014 in der Ophthalmologie einsetzbar sein. Dazu muss sie der Norm genügen, so dass das Design der LED einige Voraussetzungen erfüllen sollte.

Zum einen darf die Verwendung der Vorrichtung der vorliegenden Erfindung keine thermischen Wechselwirkungen hervorrufen, die eine Schädigung des Okulargewebes, insbesondere der sehr empfindlichen Netzhaut, verursachen. Die thermischen Wechselwirkungen beruhen prinzipiell auf unspezifischer Absorption von Photonen und resultieren in einer Erwärmung des Gewebes. Eine thermische Netzhautschädigung ist zum Beispiel dann zu erwarten, wenn durch Absorption von optischer Strahlung eine Temperaturerhöhung von 10 bis 20 °C im retinalen Pigmentepithel erreicht wird. Eine thermische Schädigung tritt nur dann auf, wenn ein Schwellenwert überschritten wird. Da jegliche Art von Energie, sei es durch den Zerfall eines angeregten elektronischen Zustandes eines Moleküls (z. B. von Sauerstoff) oder durch direkte Absorption der einfallenden Strahlung, die Temperatur erhöhen kann, ist die Expositionszeit und die Intensität der Strahlung entscheidend dafür, ob eine Schädigung eintritt.

Deshalb muss zum einen die von der Lichtquelle erzeugte thermische Belastung weniger als 0,7 W/cm$^2$ sein. Gemessen wird die thermische Belastung gemäß DIN EN ISO 15004-2:2014 radiometrisch unter Berücksichtigung der maximalen Intensität der Abstrahlcharakteristik. Bei der Ausführungsform, bei der die Lichtquelle zumindest zum Teil in den Augeninnenraum insertiert wird, wird die Bestrahlung in 18 mm Abstand zwischen Kontaktfläche des Gehäuses der Lichtquelle und der Retina bewertet (Innendurchmesser Auge + Sklera + Aderhaut), wobei von dem Fall ausgegangen wird, bei dem der kürzeste Abstand entsteht, d.h. die Sklera durch Druck verdünnt ist.

Bei der Ausführungsform, bei der die Lichtquelle lediglich von außen an die Sklera gepresst wird, wird nicht unbedingt am Pars plana beleuchtet. Wird unterhalb der Pars plana beleuchtet, so ist der Abstand zur Retina im ungünstigsten Fall nur 1 mm.

Außerdem darf durch die von der Lichtquelle erzeugte thermische Belastung der Augeninnenraum um nicht mehr als 10 - 20 °C erwärmt werden.

Zum anderen darf die von der Lichtquelle abgegebene Strahlung keine photochemische Wechselwirkung mit dem Okulargewebe, insbesondere der Netzhaut, verursachen, die phototoxische Schädigungen in dem Gewebe auslösen, die ein Ausmaß haben, das die Vorrichtung zur Verwendung in der Ophthalmologie gemäß DIN EN ISO 15004-2:2014 ungeeignet macht.

Photochemische Wechselwirkungen sind Vorgänge bei denen die Energie der einfallenden Strahlung nicht in Wärme, sondern in chemische Reaktionsenergie umgewandelt wird. Besonders im kurzwelligen Bereich, unter 400 nm Wellenlänge dominieren diese Effekte, da die Photonenenergie bei dieser Wellenlänge groß genug ist, um kovalente Bindungen zu spalten.

Das Ausmaß der phototoxischen Schädigung hängt außer von dem Spektrum und der Intensität der Strahlung auch von der Expositionszeit ab. Zur Bestimmung der maximalen Expositionszeit ist ein Grenzwert von 10 J/cm$^2$ festgelegt. Dieser dient dazu ein maximales Maß an emittierter Strahlung festzulegen, die die Netzhaut ohne Schäden übersteht. Eine maximale Expositionszeit von mindestens 10 Minuten, oder bevorzugt von mindestens 60 Minuten ist bevorzugt, da innerhalb dieser Zeitintervalle die diagnostischen, chirurgischen, oder therapeutischen Verfahren, die einer Ausleuchtung des intraokularen Raumes bedürfen, in der Regel längst abgeschlossen sind.

Das Spektrum der in der Vorrichtung der vorliegenden Erfindung verwendeten Lichtquelle darf keine detektierbare Strahlung mit einer Wellenlänge von weniger als 400 nm aufweisen. Dadurch wird sichergestellt, dass die Phototoxizität der durch die Lichtquelle abgegebenen Strahlung auf der Retina des Auges gesenkt wird. Außerdem wird dadurch gewährleistet, dass eine maximale Expositionszeit $t_{max}$ gemessen gemäß DIN EN ISO 15004-2:2014 in einem Abstand von 18 mm und 1 mm zwischen Kontaktfläche des Gehäuses und der Netzhaut des menschlichen oder tierischen Auges durch die Vorrichtung der vorliegenden Erfindung zur Verfügung gestellt ist, die eine vollständige Durchführung der diagnostischen, chirurgischen, oder therapeutischen Verfahren ermöglicht.

Durch die oben beschriebenen Parameter wird sichergestellt, dass mit hoher Wahrscheinlichkeit keine phototoxischen Schädigungen des Okulargewebes auftreten, da die für die photochemischen Wechselwirkungen verantwortliche kurzwellige Strahlung fehlt. Außerdem wird durch die Auswahl des Materials, aus dem der optionale Diffusorbereich des Gehäuses hergestellt ist, und die damit erhaltene Streuung der von der Lichtquelle abgegebenen Strahlung durch den und dadurch auch in dem bestrahlten Okulargewebe sichergestellt, dass es nicht zu einer Fokussierung der Strahlung kommt, und damit zu lokal gesteigerten Strahlungsintensitäten, die thermische oder photochemische Wechselwirkungen mit dem bestrahlten Gewebe auslösen, die das Gewebe schädigen können.

Die erfindungsgemäße Vorrichtung weist auch eine wie in den Ansprüchen definierte Fixiervorrichtung auf, die die Vorrichtung am oder im Auge so fixiert, dass der intraokulare Raum beleuchtet wird, ohne dass der Chirurg die Lichtquelle halten muss. Die Fixiervorrichtung kann durch die die Kontaktfläche (16) des Gehäuses (1) an der äußeren Oberfläche der Sklera (4) des zu beleuchtenden Auges vorübergehend während der Verwendung durch Pressen an die Oberfläche der Sklera des Auges einstellbar und fixierbar sein. Die stabile Anbringung der Vorrichtung auf der Oberfläche der Sklera des menschlichen oder tierischen Auges wird in diesem Fall durch Ausüben und Aufrechterhalten eines Anpressdrucks zwischen der Kontaktfläche des Gehäuses und der Oberfläche ermöglicht. Da die erfindungsgemäße Vorrichtung durch ihre Ausgestaltung stabil an dem Auge anbringbar ist, ist das Halten der Vorrichtung durch eine Person nicht erforderlich. Deshalb ist es dem Anwender der Vorrichtung der vorliegenden Erfindung ermöglicht, während der Verwendung der erfindungsgemäßen Vorrichtung beide Hände zur Verfügung zu haben. Da die Fixiervorrichtung für eine stabile Anbringung der Vorrichtung am zu beleuchtenden Auge sorgt, wird auch sichergestellt, dass die Beleuchtungsintensität im intraokularen Raum nicht durch eine ungewollte Bewegung der Vorrichtung verändert wird.

In einer bevorzugten Ausführungsform weist die erfindungsgemäße Vorrichtung eine Fixiervorrichtung auf, durch die sie, zum Beispiel, an einer außen am Auge eingesetzten Vorrichtung, wie etwa einem Lidsperrer, anbringbar ist. Hierzu kann die Vorrichtung mit dem Lidsperrer über Aufsätze, Klemmen, Schraubverbindungen, Klebeverbindungen, oder ähnliche Mittel verbindbar sein. Vorzugsweise ist die Verbindung leicht und schnell herzustellen und zudem reversibel. Dies gibt den Vorteil, dass die Vorrichtung, die zum Beispiel als bereits sterilisiertes Einmalprodukt vertrieben werden kann, unmittelbar vor der Anwendung leicht und einfach mit dem Lidsperrer verbunden werden kann, und nach Beendigung der Anwendung leicht und einfach wieder von dem Lidsperrer entfernt werden kann.

In einer bevorzugten Ausführungsform weist die erfindungsgemäße Vorrichtung einen Aufsatz auf, der mit dem Gehäuse im Kontakt steht und, zum Beispiel, an einem Lidsperrer anbringbar ist. Zur Anbringung an dem Lidsperrer kann der Aufsatz eine oder mehrere Aussparungen aufweisen, in die Teile des Lidsperrers eingeschoben werden können, wodurch die Anbringung des Aufsatzes an dem Lidsperrer fixierbar ist (siehe Figuren 4 und 5).

In einer bevorzugten Ausführungsform weist die erfindungsgemäße Vorrichtung eine Fixiervorrichtung auf, die so ausgestaltet ist, dass das Gehäuse so an der außen am Auge eingesetzten Vorrichtung, wie etwa einem Lidsperrer, anbringbar ist, dass die Position des Gehäuses relativ zur Position der außen am Auge eingesetzten Vorrichtung, wie etwa eines Lidsperrers, in Richtung zu der Sklera des menschlichen oder tierischen Auges hin oder von der Sklera weg einstellbar ist, wenn der Lidsperrer bestimmungsgemäß an dem Auge angebracht ist, wodurch der Anpressdruck zwischen der Kontaktfläche des Gehäuses und der äußeren Oberfläche der Sklera einstellbar ist. So kann vor der Verwendung der erfindungsgemäßen Vorrichtung die Vorrichtung durch Verbinden des Aufsatzes mit dem Lidsperrer an dem Lidsperrer angebracht werden, worauf der Lidsperrer bestimmungsgemäß eingesetzt werden kann, um die Lider des zu beleuchtenden Auges zurückzuhalten. Dann kann durch Veränderung der relativen Position des Gehäuses im Bezug auf den in einer fixen Position mit dem Lidsperrer verbundenen Aufsatz die Kontaktfläche des Diffusorbereiches des Gehäuses in Kontakt mit der Sklera des zu beleuchtenden Auges gebracht werden.

In einer bevorzugten Ausführung steht das Gehäuse (1) mit dem Aufsatz (10) über einen Gewindegang (11) in Kontakt, der auf der Außenseite eines röhrenförmigen Gehäuses angebracht ist. Der Gewindegang ist so ausgestaltet, dass er in eine Aussparung greifen kann, die in dem Aufsatz angebracht ist. Durch Drehen des Gehäuses um seine Längsachse kann somit das Gehäuse in Bezug zur Position des Aufsatzes in axialer Richtung bewegt werden. Die Ganghöhe des Gewindeganges und der Rotationswinkel bestimmen dabei das Ausmaß der Positionsveränderung. In einer bevorzugten Ausführungsform ist der Gewindegang so ausgestaltet, dass maximal eine Rotation des Gehäuses in Längsrichtung um 180° möglich ist, da Rotationen mit höheren Winkeln zu große Torsionskräfte auf die mit dem Gehäuse verbundenen elektrischen Leitungen ausüben könnten. Außerdem ist der Gewindegang so ausgestaltet, dass seine Ganghöhe für das erforderliche Ausmaß der möglichen axialen Positionsveränderung des Gehäuses sorgt. Somit ist eine bevorzugte Ausführungsform der Fixiervorrichtung so ausgestaltet, dass die Position des Gehäuses relativ zur Position der außen am Auge eingesetzten Vorrichtung, wie etwa des Lidsperrers, in Richtung zu der Sklera des menschlichen oder tierischen Auges hin oder von der Sklera weg mittels Drehung des Gehäuses einstellbar ist, wenn die außen am Auge eingesetzte Vorrichtung, wie etwa der Lidsperrer, bestimmungsgemäß an dem Auge angebracht ist, wobei das Gehäuse z.B. röhrenförmig ist, und axial in Richtung des zu beleuchtenden intraokularen Raumes ausgerichtet ist, und einen Gewindegang auf der äußeren Oberfläche aufweist, wobei der Gewindegang über eine in die Fixiervorrichtung eingebrachte Aussparung mit der Fixiervorrichtung in Verbindung steht, wobei die Fixiervorrichtung mit der außen am Auge eingesetzten Vorrichtung, wie etwa dem Lidsperrer, in einer fixen Position verbindbar ist.

Wie oben beschrieben wird durch die Fixiervorrichtung eine stabile Anbringung der Vorrichtung auf der äußeren Oberfläche der Sklera des menschlichen oder tierischen Auges durch Ausüben und Aufrechterhalten eines Anpressdrucks zwischen der Kontaktfläche des Gehäuses und der Augenoberfläche ermöglicht. Durch die besonderen Eigenschaften der Sklera ist es zudem möglich, durch Erhöhung des Anpressdrucks zwischen Kontaktfläche und Sklera die Beleuchtungsintensität im intraokularen Raum zu steigern.

Die Sklera ist ein Gewebe, das zum größten Teil aus Wasser und Kollagen besteht. Weitere Bestandteile sind nicht kollagene Proteine und Mucopolysaccharide, Lipide sowie anorganische Substanzen.

Die Streuung der optischen Strahlung ist in der Sklera sehr hoch, was eine Folge der unterschiedlichen Brechungsindizes der inhomogenen Kollagenfibrillen (n = 1,47) und der die Kollagenfibrillen umgebenden Grundsubstanz (n = 1,36) ist. Der Durchmesser der Fibrillen variiert zwischen 30 nm und 300 nm, mit einem Durchschnitt von 60 nm. Die Variation der Durchmesser ist die Ursache für die sehr starke Variation des Brechungsindex der Sklera und somit auch der Grund für die wellenlängenabhängige Streuung.

Die Transmission in der Sklera kann gesteigert werden, indem Druck auf die Sklera ausgeübt wird. Die dadurch verursachte Verdünnung der Sklera und somit Reduktion des Abstandes zwischen den Kollagenfibrillen führt zu einer Modifikation der Interferenz des durch die angrenzenden Fibrillen gestreuten Lichts. Entweicht hier mehr Wasser als Proteine und Mucopolysaccharide steigt die Konzentration der Grundsubstanz und der Brechungsindex verändert sich in Richtung des Brechungsindex der Kollagenfibrillen. Durch diesen Effekt wird die Streuung im Gewebe reduziert, die Transmission steigt und der Abstrahlwinkel hinter der Sklera verkleinert sich. Je stärker der Druck umso stärker ist dieser Effekt.

Deshalb umfasst die vorliegende Erfindung auch eine wie oben beschriebene Vorrichtung der vorliegenden Erfindung zur Steigerung der Helligkeit in dem ausgeleuchteten intraokularen Raum mittels Steigerung des Anpressdrucks.

In dieser bevorzugten Ausführungsform ermöglicht die wie oben beschriebene Fixiervorrichtung eine Veränderung der relativen Position der Kontaktoberfläche des Gehäuses bezogen auf die Position des mit dem Lidsperrer verbundenen Aufsatzes, in Richtung zu der Sklera des menschlichen oder tierischen Auges hin oder in Richtung von der Sklera weg, wenn der Lidsperrer an dem Auge angebracht ist, wodurch der Anpressdruck zwischen der Kontaktfläche des Gehäuses und der Lederhaut variiert werden kann. Bei einer Positionsveränderung, die die Kontaktoberfläche des Gehäuses in Richtung zu der Sklera hin verschiebt, wird der Anpressdruck erhöht, was wie oben beschrieben zu einer Steigerung der Helligkeit in dem ausgeleuchteten intraokularen Raum führt. Umgekehrt wird die Helligkeit in dem ausgeleuchteten intraokularen Raum verringert, wenn die Kontaktoberfläche des Gehäuses in Richtung von der Sklera weg verschoben wird, was den Anpressdruck senkt.

Diese Variabilität und Anpassungsmöglichkeiten verleihen der erfindungsgemäßen Vorrichtung den Vorteil, dass leicht und einfach, sowie auch nur vorübergehend, eine Justierung der Helligkeit im ausgeleuchteten intraokularen Raum vorgenommen werden kann, indem die Position der Vorrichtung auf dem Lidsperrer verändert wird. Zum Beispiel kann bei einem diagnostischen Verfahren oder einem Behandlungsverfahren kurzzeitig eine intensivere Ausleuchtung erwünscht sein, die dann durch eine vorübergehende Veränderung der Position der Kontaktoberfläche des Gehäuses relativ zur der Position des Lidsperrers in Richtung zu der Sklera des menschlichen oder tierischen Auges hin, was den Anpressdruck steigert, bereitgestellt wird.

Die Veränderung der Position der Kontaktoberfläche des Gehäuses relativ zur der Position des Lidsperrers kann folgendermaßen erfolgen. Nachdem wie oben beschrieben die Kontaktfläche des Gehäuses mit der Oberfläche der Sklera in Kontakt gebracht wurde, kann durch weitere Rotation des Gehäuses, die für eine Veränderung der relativen Position des Gehäuses bezogen auf die mit dem Lidsperrer verbundene Fixiervorrichtung in Richtung der Sklera sorgt, der Anpressdruck auf die Sklera erhöht werden. Eine Rotation in die entgegengesetzte Richtung sorgt somit für eine Senkung des Anpressdrucks, da die Kontaktoberfläche von der Sklera wegbewegt wird, jedoch ohne den Kontakt mit der Oberfläche der Sklera zu verlieren. Somit ist durch Rotation des Gehäuses um seine Längsachse eine Anpassung der Beleuchtungsintensität im zu beleuchtenden intraokularen Raum möglich.

Deshalb umfasst die vorliegende Erfindung auch ein Verfahren zur Anpassung der Beleuchtungsintensität in einem intraokularen Raum durch Anpassung des Anpressdrucks zwischen der Kontaktfläche der erfindungsgemäßen Vorrichtung und der Sklera des zu beleuchtenden menschlichen oder tierischen Auges.

In einer alternativen Ausführungsform ist die Fixiervorrichtung so ausgestaltet, dass der Diffusorbereich des LED-Gehäuses eine Lichtsonde aufweist, die eine geometrische Form hat, zum Beispiel eine wie in Figur 2 gezeigte konische Form, oder eine wie in Figur 7 gezeigte Form, die zur transkonjunktivalen Insertion geeignet ist, wobei sich der Durchmesser der Lichtsonde vom Gehäuse in Richtung von dem Gehäuse weg erhöht, und wobei der maximale Außendurchmesser am distalen Ende der Lichtsonde 20 G ist (siehe Figuren 2 und 6).Die untere Grenze ist nur durch die physikalischen Gegebenheiten begrenzt. Die Einheit G steht für die Feinheit des Außendurchmessers. Je höher der Gauge-Wert, desto geringer ist der Außendurchmesser der Lichtsonde. Kommerziell sinnvoll sind derzeit Außendurchmesser bis zu 29G. In bevorzugten Ausführungsformen liegt der Außendurchmesser in einem Bereich von etwa 20 bis etwa 29 G.

In dieser Ausführungsform kann der Insertionsbereich trokarartig ausgestaltet sein, um, geeigneter Weise im Bereich der Pars Plana, insertiert werden und lässt eine direkte intraokulare Beleuchtung zu. Die Beleuchtung mit dieser Vorrichtung kann zwei Anteile an Licht aufweisen, zum einen das direkt von der Lichtquelle in den intraokularen Raum abgegebene Licht und einen Lichtanteil von der Kontaktfläche der LED, der über die Sklera einfällt. Nach Insertion der Lichtsonde in den intraokularen Raum des zu beleuchtenden Auges wird die Kontaktfläche des Diffusorbereichs des Gehäuses von außen an die Sklera gepresst und in dieser Position durch die geometrische Form der Lichtsonde gehalten, da der Konus eine Bewegung der Vorrichtung von der Sklera weg verhindert. Die Lichtsonde dient hiermit nicht nur der direkten Beleuchtung des intraokularen Raumes, sondern auch als Fixiervorrichtung. In einer weiteren Ausführungsform weist die gesamte LED eine zur transkonjunktivalen Insertion geeignete geometrische Form auf, so dass die gesamte LED, und nicht nur ein zur Lichtsonde abgedrehter Teil des LED-Gehäuses, insertiert und in der Sklera verankert werden kann.

In einer weiteren Ausführungsform weist die erfindungsgemäße Vorrichtung sowohl die oben beschriebene Fixiervorrichtung in Verbindung mit dem Lidsperrer, als auch die wie oben beschrieben Fixiervorrichtung in Form der insertierbaren konisch geformten Lichtsonde auf. In dieser Ausführungsform ist der Vorteil der Kombination der transskleralen und der direkten Beleuchtung mit der Möglichkeit, die Intensität der Beleuchtung mittels Erhöhung des Anpressdruckes zwischen Vorrichtung und Sklera kombiniert. Diese Vorrichtung ermöglicht dem Chirurgen eine Feineinstellung der Beleuchtungsintensität während des Eingriffes.

Das Gehäuse, das die Lichtquelle umgibt, ist vorzugsweise durch ein Spritzgussverfahren hergestellt. Vorzugsweise ist das Material, aus dem das Gehäuse hergestellt ist, ein Polymer, das eine Temperatur, wie sie beim Autoklavieren angewendet wird, ohne Verformung aushält. Es kann z.B. ein Duroplast sein, wie etwa Acrylnitril-Butadien-Styrol (ABS), dessen Glasumwandlungstemperatur oberhalb von 121 °C liegt, was eine effektive, problemlose Sterilisierung, wie etwa durch Autoklavieren, ermöglicht. Obwohl die Vorrichtung für die einmalige Verwendung geeignet ist, sorgt doch die Auswahl der verwendeten Materialen für die Möglichkeit der Wiederverwendung, da die Materialien sterilisierbar sind. Darüber hinaus ermöglicht die Auswahl der Materialien eine Sterilisierung nach Zusammensetzung der Einzelteile der Vorrichtung, wodurch sich die Vorrichtung in steriler Weise verpackt vertreiben lässt. So wird ein Zusammenbau der Vorrichtung unmittelbar vor der Anwendung, was eine mögliche Kontaminationsquelle darstellt, überflüssig.

In einer weiteren bevorzugten Ausführungsform der Vorrichtung der vorliegenden Erfindung ist die von der Lichtquelle, wie etwa einer LED oder OLED, abgegebene Strahlung seitlich im Bezug auf die Linie, die den Strahlungsausgang der Lichtquelle mit dem zu beleuchtenden intraokularen Raum verbindet, abgeschirmt. Damit wird zum einen eine mögliche Blendung des Verwenders der erfindungsgemäßen Vorrichtung, zum Beispiel medizinisches Personal wie etwa ein Ophthalmologe, reduziert. Zum anderen kann dadurch die Beleuchtungsintensität im intraokularen Raum erhöht werden.

Die seitliche Abschirmung kann mit jedem hierzu geeigneten Material erfolgen. Geeignet zur seitlichen Abschirmung ist zum Beispiel ein opaker, lichtundurchlässiger Überzug. In einer bevorzugten Ausführungsform dient eine opake, blickdichte, schwarze Hülle um die Lichtquelle und das Gehäuse, die den Diffusorbereich des Gehäuses nicht einschließt, zur seitlichen Abschirmung. Diese Hülle kann entweder als tatsächliche auch mechanisch nicht transparente Hülle oder in Form zum Beispiel einer zusätzlichen lichtundurchlässigen, intransparenten, zum Beispiel einer schwarzen Lackschicht ausgestaltet sein. Eine weitere bevorzugte Ausführungsform weist eine nicht transparente Schicht oder Hülse auf, die den Diffusorbereich des Gehäuses nicht einschließt, und die zumindest in ihrem Inneren metallisch oder weiß oder anderweitig hoch reflektierend ausgestaltet ist. Dadurch wird die Intensität der optischen Strahlung an der Kontaktfläche des Diffusorbereichs mit dem Auge erhöht. Eine weitere bevorzugte Ausführungsform weist ein Gehäuse in Kegelform auf, wobei sich der Kegel in Richtung zum Auge hin verbreitert. Diese Form hat zur Folge, dass Lichtstrahlen im Inneren des Gehäuses mit größeren Winkeln zur Normalen auf der Gehäusefläche auftreffen. Damit werden mehr Lichtstrahlen in eine Richtung zum Auge hin totalreflektiert. Die Verluste zur Seite und damit auch die Blendung des Chirurgen werden geringer und der Lichtstrom zum Auge hin erhöht sich.

In der Ausführungsform, die eine LED aufweist, ist die LED-Fassung bevorzugt durch ein Spritzgussverfahren hergestellt. Am meisten bevorzugt ist eine LED-Fassung, die aus einem transparenten augenverträglichen Polymer, wie Polymethylmethacrylat (PMMA; Plexiglas) hergestellt ist, da die Streuung in PMMA variabel ist, und so diffuses Licht emittiert werden kann. Zudem hat PMMA keine reizende Wirkung und ist somit nicht als Gefahrstoff deklariert Deshalb eignet sich ein augenverträgliches transparentes Polymer wie PMMA zur Verwendung in ophthalmologischen Verfahren.

Ein Vorteil der Verwendung einer LED ist es, dass die Farbtemperatur einstellbar ist. In einer bevorzugten Ausführungsform hat die Lichtquelle ein Spektrum, das eine Farbtemperatur von etwa 2.600 K bis etwa 3.500 K hat, was auch als "warmweiße" Farbtemperatur bezeichnet wird, wobei die CIE-Koordinaten von 0,35 - 0,45(x)/0,35 - 0,45(y) sind, da "warmweißes" Licht einen natürlichen Farbeindruck hervorruft und die Wahrnehmung, insbesondere die farbliche Wahrnehmung, nicht verfälscht. In einer noch bevorzugteren Ausführungsform hat die LED ein Spektrum, das die CIE-Koordinaten von etwa 0,42(x)/0,41(y) hat, was ein leicht ins gelbliche verschobenes "warmweiß" ist, das aber keinen starken Gelbstich aufweist, der die Wahrnehmung verfälscht. In anderen Ausführungsformen kann der Blauanteil erhöht sein, damit die Beleuchtung nicht zu rötlich wirkt. Der verwendete Farbton der LED kann auch abhängig von der verwendeten Vorrichtung eingestellt werden, z.B. danach ob nur Licht über die Sklera einfällt oder ob direkt intraokular beleuchtet wird.

In weiteren Ausführungsformen, besonders in Vorrichtungen zur Verwendung in diagnostischen Verfahren kann es vorteilhaft sein, anstatt der warmweißen LED eine einfarbige oder mehrfarbige LED oder OLED oder Kombination daraus zu verwenden. Die erfindungsgemäße Vorrichtung lässt es zu, LED oder OLED entsprechend auszuwählen. So kann bei Verwendung eines Farbstoffs zum Anfärben von Strukturen im Auge durch Auswahl einer LED oder OLED mit geeigneter Wellenlänge bzw. Farbtemperatur der Farbstoff intensiviert werden und die Sichtbarkeit erhöht werden. Die LED oder OLED können alle gleich sein oder es kann durch geeignete Kombinationen ein gewünschter Farbeindruck erzeugt werden.

Außerdem kann die Vorrichtung der vorliegenden Erfindung auch eine Vielzahl an LED aufweisen, die so in dem Gehäuse angeordnet sind, dass deren abgegebene Strahlung über die gesamte Fläche der Kontaktfläche gleichmäßig verteilt ist, was zu einer gleichmäßigen Ausleuchtung beiträgt. In bevorzugten Ausführungsformen sind die LED organische LED (OLED), auch als Flächenstrahler bezeichnet, welche flexibel sind, wodurch sich die thermischen, photochemischen, und mechanischen Belastungen über eine größere Fläche verteilen lassen.

In weiteren Ausführungsformen ist die Kontaktfläche der Vorrichtung mit der Augenoberfläche vergrößert, indem gekrümmte Kontaktflächen verwendet werden, deren Krümmung der Augenoberfläche entspricht, so dass die gesamte Kontaktoberfläche der erfindungsgemäßen Vorrichtung an der Oberfläche des Auges anliegen kann. Die Vergrößerung der Kontaktoberfläche sorgt durch Verteilung des Druckes und der abgegebenen Strahlung über die gesamte Kontaktoberfläche zu einer weiteren Verringerung des Risikos, das Okulargewebe zu schädigen.

Die für die erfindungsgemäße Vorrichtung benötigte Spannung kann der Lichtquelle in üblicher Weise zugeführt werden. Bevorzugt ist die Spannung Gleichspannung und wird durch eine Knopfzelle oder einen Kondensator geliefert. Dies ermöglicht eine miniaturisierte Elektronik, deren Energiequelle die Lichtquelle mehrere Stunden lang versorgt. Diese miniaturisierte Energiequelle kann auch leicht während der Verwendung z.B. an der Stirn des Patienten befestigt werden. In einer Ausführungsform weist die Spannungsquelle ein Gehäuse auf, das einen selbstklebenden Bereich aufweist, mit dem die Vorrichtung zum Beispiel an der Stirn des Patienten befestigt werden kann. Zudem weist das Gehäuse bevorzugt einen Spalt auf, der einen kontaktblockierenden Streifen aufweist. Nach Entfernung dieses Streifens wird der Kontakt wieder hergestellt und der Stromkreis geschlossen, was die Lichtquelle, zum Beispiel die LED zum Leuchten bringt. Diese Ausführungsform stellt eine preiswerte Variante im Vergleich zu bestehenden Beleuchtungssystemen dar, und eignet sich deshalb als Einmalprodukt, das also nach einmaliger Verwendung entsorgt werden kann. Dies ist vor allem bei medizinischen Geräten von Nutzen, wo vor Wiederverwendung zumindest eine Sterilisation möglich sein muss. Aufgrund der preiswerten Bauweise ist die Vorrichtung der vorliegenden Verwendung als Einmalprodukt geeignet.

Somit stellt die vorliegende Erfindung eine Verwendung einer Ausführungsform der Vorrichtung zur nicht invasiven gleichmäßigen Beleuchtung des intraokularen Raumes eines menschlichen oder tierischen Auges zur Verfügung. Die Beleuchtung erfolgt aufgrund der oben dargelegten Vorteile transskleral, das heißt, dass die Oberfläche des Auges, die in Kontakt mit dem Gehäuse steht, die Sklera ist. Ein weiterer Vorteil dieser Ausführungsform der vorliegenden Erfindung ist die nicht-Invasivität der Verwendung, die durch die Ausgestaltung der erfindungsgemäßen Vorrichtungen ermöglicht ist. Das bedeutet, dass bei der Verwendung weder die Sklera noch andere Gewebe des Auges durchstoßen werden. Durch die nicht-invasiven und deshalb gewebeschonenden Ausführungsformen der vorliegenden Erfindung wird auch ausgeschlossen, dass es selbst bei einer Fehlbedienung zu Schäden kommt, im Gegensatz zu Vorrichtungen des Stands der Technik, bei denen zum Beispiel in den Augeninnenraum insertierte Lichtleiter für die Ausleuchtung verwendet werden. Deshalb ist die erfindungsgemäße Verwendung zur nicht-invasiven gleichmäßigen Beleuchtung des intraokularen Raumes eines menschlichen oder tierischen Auges bevorzugt eine diagnostische Verwendung, wobei die Integrität des Okulargewebes, zum Beispiel der Netzhaut, überprüft wird, ohne einen chirurgischen Eingriff vorzunehmen.

Die Ausführungsform, bei der die Vorrichtung eine Lichtsonde zur Insertion in den intraokularen Raum aufweist, ist durch die Kombination der direkten und der transskleralen Beleuchtung des intraokularen Raumes vorteilhaft. Diese Ausführungsform der vorliegenden Erfindung nutzt die optischen Eigenschaften der Sklera, beleuchtet aber zusätzlich den intraokularen Raum mit einem insertierten Lichtleiter. Sie kombiniert dadurch die gleichmäßige Ausleuchtung mit einem sehr hellen Lichtkegel, was eine bis in die Peripherie helle und gleichmäßige Ausleuchtung erzeugt. Zudem eignet sich diese Vorrichtung bevorzugt für Verwendungen, bei denen ein Eingriff in das Okulargewebe ohnehin erforderlich ist, wodurch die invasive Maßnahme der Insertion der Lichtsonde keinen zusätzlichen Nachteil darstellt. Deshalb ist die erfindungsgemäße Verwendung, die die transsklerale mit der direkten Beleuchtung kombiniert, bevorzugt eine chirurgische Verwendung, wo durch den Eingriff bereits eine Invasivität gegeben ist.

Bevorzugt wird transskleral über die Pars-Plana-Region der Sklera beleuchtet, da in diesem Bereich der Schaden durch eine eventuelle Insertion relativ gering ist, da kein spezialisiertes, funktionell wichtiges Gewebe vorhanden ist. Die Beleuchtung kann jedoch auch an weiter seitlich oder weiter hinten gelegenen Bereichen des Auges, wie etwa der Augenrückseite erfolgen.

Die Vorrichtungen, Verwendungen oder Verfahren der vorliegenden Erfindungen dienen bevorzugt zur Beleuchtung des intraokularen Raumes eines menschlichen Auges. Es können jedoch auch die Augen eines Schweines, Pferdes, Hundes, einer Katze oder eines anderen Säugetiers beleuchtet werden.

Die erfindungsgemäße Vorrichtung eignet sich zur Verwendung in chirurgischen und diagnostischen Verfahren am Auge.

Die Erfindung wird auch durch die folgenden Beispiele, die Vorrichtungen und Verwendungen, und Verfahren, sowie Bestandteile derjenigen beschreiben, erläutert, ohne sie darauf zu beschränken.

**Bezugszeichenliste**

(1)   Gehäuse
(2)   Lichtquelle (LED, OLED)
(3)   Lichtsonde
(4)   Sklera
(5)   Streuung im Gewebe
(6)   Intraokularer Raum
(7)   Auge
(8)   Lichtleiter
(9)   Spektrometer
(10)  Aufsatz
(11)  Gewindegang
(12)  Lidsperrer
(13)  Gehäuse um Spannungsquelle
(14)  Elektrischer Leiter
(15)  Spannungsquelle; Knopfzelle
(16)  Kontaktfläche des Gehäuses

**Beispiele**

Beispiel 1: Bewertung nach DIN EN ISO 15007-2:2014

Maßgeblich für die Dimensionierung und Klassifizierung der Vorrichtung zur Beleuchtung des intraokularen Raumes (auch als Endoilluminator bezeichnet) ist die Norm DIN EN ISO 15004-2:2014. Sie beinhaltet eine Bewertung der photochemischen Gefährdung durch Licht für die Netzhaut des aphaken Auges. Die Bestrahlungsstärke $E_{A-R}$ des Endoilluminators, wird über den Spektralbereich zwischen 305 und 700 nm, der jeweiligen Lichtquelle, integriert. Die spektrale Bestrahlungsstärke $E_\lambda$ wird hierfür mit $\Delta\lambda$ und mit einer Bewertungsfunktion $A(\lambda)$ für die Gesamt-Phototoxizität (Figur 3A), die bei niedrigen Wellenlängen zunimmt, multipliziert. $A(\lambda)$ berücksichtigt auch den "Blue light hazard", der bei Wellenlängen zwischen 400 und 500 nm sein Maximum hat. Aus der Bestrahlungsstärke $E_{A-R}$ wird dann die mögliche Expositionszeit $t_{max}$ berechnet. Zur Bestimmung der maximalen Expositionszeit ist ein Grenzwert von 10 J/cm² festgelegt. Dieser dient um ein maximales Maß an emittierter Strahlung festzulegen, die die Netzhaut ohne Schäden übersteht. Der Grenzwert der Bestrahlungsstärke $E_{A-R}$ ist für die Einstufung in die Gefahrengruppe 1 auf 440 μW/cm², deklariert.

Bestrahlungsstärke $E_{A-R}$:

$$E_{A-R} = \sum\nolimits_{305}^{700} E_\lambda * A(\lambda) * \Delta\lambda \qquad (1)$$

Maximale Expositionsdauer $t_{max}$:

$$t_{max}(E_{A-R}) = \frac{10\,\frac{J}{cm^2}}{\sum_{305}^{700} E_\lambda * A(\lambda) * \Delta\lambda} \qquad (2)$$

Durch Mittelwertbildung der höchsten lokalen Strahlungsleistung auf der Netzhaut, die auf eine kreisförmige Fläche mit einem Durchmesser von 0,18 mm ($2,52*10^{-4}$ cm²), bei einem ruhiggestellten Auge von 0,03 mm ($7,07*10^{-6}$ cm²), trifft, wird die Bestrahlungsstärke beurteilt. Emittiert die Quelle einen gleichförmigen Strahl von durchschnittlich mehr als 1 mm Durchmesser, darf eine kreisförmige Fläche mit einem Durchmesser von 1 mm genommen werden. Aus messtechnischen Gründen wurde der Durchmesser von 1 mm gewählt, was aber keinen Nachteil mit sich bringt, da es zu höheren Ergebnissen und somit auch zu einem größeren Maß an Sicherheit gegenüber der photochemischen Gefährdung auf der Netzhaut führt.

Für die Gefährdung durch Erwärmung durch sichtbare und infrarote Strahlung auf der Netzhaut, wird die thermische Bestrahlungsstärke $E_{VIS-R}$ berechnet. Sie wird über den Spektralbereich von 380 bis 1400 nm integriert. Die spektrale Bestrahlungsstärke $E_\lambda$ wird hierfür mit $\Delta\lambda$ und einer Bewertungsfunktion $R(\lambda)$ (Figur 3B) multipliziert. $R(\lambda)$ stellt die Bewertungsfunktion der Gefährdung durch Erwärmung dar. Der für die Einstufung in die Gefahrengruppe 1 nicht zu überschreitende Grenzwert ist 0,7 W/cm².

Bestrahlungsstärke $E_{VIS-R}$:

$$E_{VIS-R} = \sum\nolimits_{380}^{1400} E_\lambda * R(\lambda) * \Delta\lambda \qquad (3)$$

Auch hier sollte der Durchmesser der kreisförmigen Fläche 0,03 mm betragen. Es wurde wiederum der Durchmesser 1 mm verwendet.

Beispiel 2: Leistungsmessungen

Da zur Messung kein Radiometer, welches über den kompletten Spektralbereich eine gesamte Strahlungsleistung ermitteln würde, zu Verfügung stand, wurden andere Messmethoden angewandt.

Die Messungen wurden mit einer Blende, mit Kreisdurchmesser d = 1 mm, vorgenommen. Es wurde im Abstand von 18 mm gemessen, da der Abstand von Augenoberfläche durch die Pupille zur Netzhaut circa 17 mm beträgt und die menschliche Sklera eine Dicke von 0,7 - 0,8 mm aufweist. Dies ist der Abstand den die Endoilluminatoren von der Retina haben.

Radiometrische Leistungsmessung

Die radiometrischen Messungen erfolgten mit dem Leistungsmessgerät Qioptiq OPM 150. Da dieses Messgerät eigentlich zur Vermessung von monochromatischen Lichtquellen vorgesehen ist, wurden nur Näherungen durch den Einsatz eines Filters gemessen. Da durch die Bewertungsfunktion $A(\lambda)$ der Wellenlängenbereich oberhalb von 500 nm vernachlässigt werden kann, wurde ein Filter eingesetzt, der den Bereich oberhalb von 500 nm abschneidet. So wurden zwei Messungen durchgeführt, einmal mit und einmal ohne Filter. Anschließend wurde die Differenz gebildet. Das Ergebnis ist die Strahlungsleistung $\Phi_e$ des blauen Peaks, welche zur Berechnung von $E_{A-R}$ ausschlaggebend ist (Abbildung 4A).

Temperaturmessung LED

Die Wärmeentwicklung auf den Oberflächen wurde durch eine Messsonde (Type K) des Temperaturmessgeräts Raytek Raynger MX4 gemessen. Die Oberfläche einer LED wurde jeweils im ursprünglichen und modifizierten Zustand (siehe Figur 2), über den Zeitraum von 60 Minuten, vermessen. Die Zeitspanne der Messung dient als Simulation der Wärmeentwicklung während einer Operation über den geplanten Zeitraum eines Einsatzes.

Messung der Abstrahlcharakteristik

Die Abstrahlcharakteristik einer LED wurde auch jeweils im ursprünglichen wie im modifizierten Zustand gemessen. Die Messung erfolgte über 180° in 10°-Intervallen und wurde in etwa 40 cm Abstand, mit dem Spektrometer Avantes SensLine AvaSpec-2048 XL, gemessen und die jeweiligen Maxima notiert. Die LED wurde senkrecht zur Detektorfläche des Lichtfilters ausgerichtet und als 0° definiert. Nun wurde von -90° bis +90° gemessen. Anschließend wurde die LED um 90° horizontal gedreht und die Messung wiederholt, um eventuelle Fertigungsungenauigkeiten der LED zu berücksichtigen.

Lichtausbeutemessung

Da bestehende Beleuchtungssysteme meist nur photometrische Angaben des emittierten Lichtstroms $\Phi_v$ der jeweiligen Lichtsonde, sowie die Lichtausbeute $\eta_v$, angeben, wurden diese Werte durch eine Ulbrichtkugel mit 1 m Innendurchmesser vermessen.

Beispiel 3: Auswahl einer geeigneten Lichtquelle

Für die korrekte Auslegung der Spezifikation der zu verwendenden Lichtquelle, und damit für die Vermeidung von durch die von der Lichtquelle abgegebenen Strahlung hervorgerufenen toxischen Effekte, ist es wichtig, die Strahlung und somit die Energie, die intraoperativ auf die Retina und auf das retinale Pigmentepithel (RPE) fällt, zu kennen. Diese hängt nicht nur von den Parametern der Lichtquelle, sondern auch von Variablen wie Einfallswinkel, Expositionsdauer und Abstand der Lichtsonde zur Retina, ab. Bei schlechten Sichtverhältnissen kann die Intensität erhöht werden, was aber extreme Auswirkungen auf die erlaubte Expositionsdauer hat.

Die potenzielle Schädigung der Retina bei intraokularer Beleuchtung wird hauptsächlich durch thermische und photochemische Wechselwirkungen ausgelöst. Die thermischen Wechselwirkungen beruhen prinzipiell auf unspezifischer Absorption von Photonen und resultieren in einer Erwärmung des Gewebes. Die photochemischen Wechselwirkungen unterscheiden sich hiervon, indem selektive Absorption stattfindet. Energiereiche Photonen des unteren Wellenlängenbereichs (< 400 nm) lösen Reaktionen aus und aktivieren weitere Prozesse im Gewebe.

Durch radiometrische Messungen der Strahlungsleistungen von LEDs wurde die Bestrahlungsstärke $E_{A-R}$, eine der Bewertungsfunktionen nach DIN EN ISO 15004-2:2014, die die photochemischen Eigenschaften durch den "Blue Light Hazard" einschließt, bestimmt. Der "Blue Light Hazard" soll möglichst gering sein, um die Auswirkungen zu minimieren. Die Phototoxizität durch photochemische Wechselwirkungen, die durch die energiereichen Photonen entstehen, soll auf einem möglichen Minimum gehalten werden. Dies spiegelt sich in der maximalen Expositionszeit $t_{max}$ wieder. Der Blauanteil der Strahlung sollte jedoch nicht zu gering sein, um einen natürlich weißen und keinen gelblichen Farbeindruck zu gewährleisten.

Die Messungen wurden in 18 mm sowie in 1 mm Abstand zwischen Kontaktfläche der Lichtquelle und der Netzhaut durchgeführt wie in den voranstehenden Beispielen spezifiziert. Dabei ist 1 mm der minimale Abstand zwischen Kontaktfläche der Lichtquelle und der Netzhaut bei der von außen angepressten Lichtquelle, wenn nicht am Pars plana sondern an einer anderen Stelle des Auges beleuchtet wird.

Die erhaltenen Ergebnisse sind durch Messung an Schweineaugen entstanden, welche zwar eine große Ähnlichkeit mit dem menschlichen Auge aufweisen, jedoch entscheidende Unterschiede haben. So unterscheiden sie sich unter anderem in Transmission, Absorption und Dicke der Sklera. Gerade durch höhere Transmission und geringere Dicke der menschlichen Sklera sind etwas höhere thermische als auch photochemische Gefährdungen zu erwarten. Durch die höhere Transmission und etwas geringere Streuung ist eine noch bessere Ausleuchtung zu erwarten.

Die Auswahlkriterien für eine geeignete Lichtquelle, wie etwa einer LED sind das Spektrum, das, wenn möglich, einen geringen Blauanteil besitzen soll, der Farbeindruck und die Größe der Lichtquelle. Der Farbeindruck "warmweiß" hat eine natürliche Farbwahrnehmung und beinhaltet geringere Blauintensitäten als "kaltweiße" LEDs. Die derzeit kleinsten, handelsüblichen LEDs haben einen Durchmesser von 1,8 mm, sind aber noch nicht mit definierten Leistungen und Farbtönen erhältlich. Der derzeit kleinste Außendurchmesser der LEDs, der eine genügende Größe an Variation bietet, ist 3 mm. Die Weiterentwicklung der LED wird in Zukunft eine weitere Miniaturisierung zulassen. Die erfindungsgemäße Vorrichtung kann dann in der Größe entsprechend angepasst werden. 3mm-LEDs sind noch nicht zu klein um sie bearbeiten zu können und bieten in Hinsicht auf Abstrahlwinkel, Farbton und Leistung ein Spektrum an Auswahl. Aus diesen Kriterien wurden 3 beispielhafte mögliche LEDs bestimmt:

LED 1:

LED 1 ist eine Flat-Top 3 mm LED mit diffusem (unregelmäßig streuendes und trübes) Gehäuse. Ein diffuses Gehäuse bringt den Vorteil mit sich, dass Licht unregelmäßig gestreut wird. Die Betriebsspannung kann von U = 2,8 bis 3,6 V variiert werden. Bei 3,6 V Spannung soll dann ein Strom von I = 20 mA fließen. Dies ergibt eine elektrische Leistung von 72 mW.

Elektrische Leistung $P_{el}$:

$$3,6\,V * 20\,mA = 72\,mW$$

Der Abstrahlwinkel ist mit 120° angegeben (kompletter Winkel) auf den sich die angegebene Lichtstärke von I = 1200 mcd bezieht. Daraus lässt sich der theoretische Lichtstrom $\Phi$= 3,77 lm abschätzen.

Raumwinkel $\Omega$:

$$4 * \pi * \left(\sin\frac{120°}{4}\right)^2 = 3,142\ \text{sr}$$

Lichtstrom $\Phi$:

$$1200\,mcd * 3,142\,sr = 3,77\,lm$$

Lichtausbeute $\eta_v$:

$$\eta_v = \frac{\Phi_v}{P_{el}} = \frac{3,77\,lm}{72\,mW} = 52,4\,\frac{lm}{W}$$

LED 2:

LED 2 ist eine 3 mm LED mit einem Gehäuse, das aus einem Material hergestellt ist, das als Lichtdiffusor wirkt. Ein solches Gehäuse bringt den Vorteil mit sich, dass das Licht unregelmäßig gestreut wird. Die Betriebsspannung kann von U = 2,8 bis 3,6 V variiert werden. Bei 3,6 V Spannung soll dann ein Strom von I = 20 mA fließen. Dies ergibt eine elektrische Leistung von 72 mW.

Elektrische Leistung $P_{el}$ :

$$3,6\,V * 20\,mA = 72\,mW$$

Der Abstrahlwinkel ist mit 40° angegeben (kompletter Winkel) auf den sich die angegebene Lichtstärke von I = 4400 mcd bezieht. Daraus lässt sich der theoretische Lichtstrom $\Phi$= 1,67 lm abschätzen.

Raumwinkel $\Omega$:

$$4 * \pi * \left(\sin\frac{40°}{4}\right)^2 = 0,379\ \text{sr}$$

Lichtstrom $\Phi$:

$$4400 \ mcd * 0,379 \ sr = 1,67 \ lm$$

Lichtausbeute $\eta_v$:

$$\eta_v = \frac{\Phi_v}{P_{el}} = \frac{1,67 \ lm}{72 \ mW} = 23,2 \ \frac{lm}{W}$$

<u>LED 3:</u>

LED 3 ist eine 3 mm LED mit transparentem Gehäuse. Die Betriebsspannung kann von U = 2,8 bis 3,6 V variiert werden. Bei 3,6 V Spannung soll dann ein Strom von I = 20 mA fließen. Dies ergibt eine elektrische Leistung von 72 mW.

Elektrische Leistung $P_{el}$:

$$3,6 \ V * 20 \ mA = 72 \ mW$$

Der Abstrahlwinkel ist mit 40° angegeben (kompletter Winkel) auf den sich die angegebene Lichtstärke von I = 9100 mcd bezieht. Daraus errechnet sich der theoretische Lichtstrom $\Phi$= 3,45 lm.

Raumwinkel $\Omega$:

$$4 * \pi * \left( \sin \frac{40°}{4} \right)^2 = 0,379 \ \text{sr}$$

Lichtstrom $\Phi$:

$$9100 \ mcd * 0,379 \ sr = 3,45 \ lm$$

Lichtausbeute $\eta_v$:

$$\eta_v = \frac{\Phi_v}{P_{el}} = \frac{3,45 \ lm}{72 \ mW} = 47,9 \ \frac{lm}{W}$$

Das Verhältnis des blauen Peaks (emittierte Strahlung des LED-Chips) zu dem breitbandigen Peak (emittierte Strahlung des fluoreszierenden Phosphors) zeigt bei LED 1 das angenehmste Farbempfinden. Die Darstellung in den CIE-Koordinaten ist laut Datenblatt 0,42(x)/0,41(y) und ergibt ein ins Gelbe verschobene Weiß, dessen Gelbstich jedoch nicht sehr stark ist.

Der blaue Peak von LED 2 ist geringer als bei LED 1. Daher erzeugt LED 2 einen sehr gelblichen Farbeindruck.

LED 3 besitzt einen dominanten Blau-Peak, der das Farbempfinden "kaltweiß"-bläulich wirken lässt, was LED 3 als nicht optimal einstuft. LED 3 emittiert keinen homogenen Farbeindruck. Die Verteilung des Phosphor auf dem LED-Chip ist in so weit inhomogen, dass die Konzentration zur Peripherie abnimmt. Sie emittiert daher im Zentrum einen blauen Lichtkegel, der in der Peripherie gelb wird.

Die Bestrahlungsstärken $E_e$ der LEDs mit modifizieren Gehäuse wurde durch die Leistungsmessungen im Abstand 18 mm photometrisch wie auch radiometrisch gemessen, um die maximale Expositionszeit $t_{max}$ zu berechnen. Die durch die photometrischen Messungen erhaltenen Beleuchtungsstärken $E_v$, ergaben bei Berechnung von $t_{max}$ einen um circa Faktor 5 größeren Wert, als die Berechnungen von $t_{max}$, mit Strahlungsfluss $\Phi_e$, welcher durch die radiometrischen Messungen erhalten wurde.

Dies ist womöglich eine Folge der Eliminierung der relativen spektralen Empfindlichkeit des Auges V($\lambda$) und veranlasste die radiometrischen Messungen als maßgeblich anzusehen, um die Sicherheit zu gewährleisten. Die in 1 mm Abstand folgenden Messungen wurden, um die mögliche Gefährdung der peripheren Retina nahe des Pars plana zu berücksichtigen, anschließend nur noch radiometrisch aufgenommen.

Für den Aufbau der Beleuchtungssysteme wurde daher LED 1 ausgewählt. Durch die in 18 mm Abstand gemessene Leistung wurden 13,40 mögliche Stunden der Bestrahlung bei einem erhöhten Strom von ca. 40 mA errechnet. Die maximalen Expositionszeiten $t_{max}$ von LED 2 (4,55 h), und LED 3 (1,84 h), entsprechen zwar auch den bevorzugten Parametern für $t_{max}$, sind jedoch weniger bevorzugt, da durch die höhere Leistung die Gefahr der Blendung des Chirurgen größer ist, und die Erfüllung der Anforderungen an das Farbempfinden und an die Messungen in 1 mm Abstand nicht optimal sind. LED 3 ist infolge der inhomogenen Emittierung von Licht weniger bevorzugt.

Aus der Vermessung der Abstrahlcharakteristik lässt sich folgern, dass die Messungen, da die Bewertung der Gefährdung immer axial bei 0° durchgeführt wird, nur circa 50 % der maximalen Intensität detektierte. Unter diesen Umständen wurden die gemessenen Werte mit Faktor zwei multipliziert, um theoretisch die maximale Intensität zu berücksichtigen. Die Ergebnisse der maximalen Beleuchtungsstärke auf der Retina ergeben dann einen rechnerischen Wert von $\Phi_{e,rech.}$ = 3,52 $\mu$W und eine Expositionszeit von circa $t_{max}$ = 6,67 h.

Die Vorrichtung zur externen transskleralen Endoillumination erzeugt eine gleichmäßige, reflexionsarme Art der intraokularen Beleuchtung. Dies ist die Folge der Streuung im Gewebe. Durch die Transmission, die bei starkem Druck und bei kurzen Wellenlängen bis zu circa 15 % beträgt und dem gestreutem Licht im Gewebe, entsteht eine gleichmäßige Beleuchtung, ohne die Bildung eines Beleuchtungskegels mit lokalem Fokus. Die geringe Transmission erzeugt eine überraschend helle Beleuchtung. Figur 1A stellt dies schematisch dar. Dies ist die Folge der großen Streuung innerhalb der Sklera, was zum einen durch die Unterschiede der Brechungsindizes von Kollagenfibrillen (n = 1,47) und Grundsubstanz (n = 1,36), zum anderen von der Beugung, die an den Kollagenfibrillen auftritt, herrührt. Diese variieren sehr stark in ihren Durchmessern, was eine große Variation der entstehenden Brechung sowie Beugung entstehen lässt und eine starke Beleuchtung des Gewebes zur Folge hat. Die Transmission und somit die Helligkeit im Intraokularraum, lässt sich durch den Druck auf die Sklera variieren. So nimmt diese bei zunehmendem Druck, durch die Verdünnung der Sklera und das Entweichen des Wassers, zu. Mit diesem zunehmenden Druck steigt die Transmission bei kurzen Wellenlängen, was zum einen eine Veränderung des Farbtons, zum anderen aber auch eine Steigerung der Gefährdung durch blaues Licht, bewirkt. Durch das Entweichen des Wassers steigt die Konzentration der Grundsubstanz und der Brechungsindex verändert sich in Richtung des Brechungsindex der Kollagenfibrillen. Durch diesen Effekt wird die Streuung im Gewebe reduziert, die Transmission steigt und der Abstrahlwinkel nach der Sklera verkleinert sich. Je stärker der Druck umso stärker ist dieser Effekt. Das Justieren des Drucks und somit auch der Helligkeit ist durch ein Gewinde möglich.

Der Unterschied der Brechungsindizes des LED-Gehäuses (n = 1,5) und des Glaskörpers (n = 1,3367) verursacht, dass der Abstrahlwinkel des transmittierten Lichts nach der Sklera kleiner ist als bei einem Übergang zur Luft. Dadurch wird eine ganzheitliche Ausleuchtung ohne einen lokalen Fokus erreicht, was eine Neupositionierung oder das Bewegen des Lichtkegels unnötig macht. Die Ausleuchtung ist immer ganzheitlich.

Die Gefährdung durch optische Strahlung, besonders durch die kuzwellige optische Strahlung, ist bei der externen Variante des LED-Endoilluminators geringer als bei einer direkten Bestrahlung. Dies hat zur Folge, dass auch die Gefährdung, durch photochemische und photodynamische Wechselwirkungen wesentlich geringer ist. Der Zerfall von Rhodopsin durch die Phototransduktion, die Bildung von freien Radikalen und die mit sich führende Bildung von aktivem Singulett-Sauerstoff wird reduziert. Auch die mögliche Gefährdung durch direkte photochemische Schädigung durch direkte Spaltung von kovalenten Bildungen nimmt ab.

Die Belastung der peripheren Retina nahe des Pars plana ist der Ort der höchsten Gefährdung. Hier trifft die transmittierte optische Strahlung im Abstand 0,7 - 0,8 mm, Dicke der menschlichen Sklera, auf die Netzhaut. Zur tatsächlichen Bewertung kann aber nicht die direkt gemessene Leistung in 1 mm Abstand $\Phi e$ = 39,08 $\mu$W genommen werden, da die Transmission sehr gering ist. Bei Annahme einer maximalen Transmission von 15 % (Messwert), bei niedrigen Wellenlängen und starkem Druck, multipliziert mit dem Faktor 2 (aufgrund der Abstrahlcharakteristik), ergibt dies eine optische Leistung von $\Phi e$ = 11,8 $\mu$W (siehe Tabelle 1).

Damit ist eine Einsatzzeit von circa zwei Stunden möglich. Diese mögliche Schädigung wird in Betracht gezogen, da die Distribution durch und die Streuung in der Sklera nicht genau bestimmt ist. Die Transmission bei 450 nm (blauer Peak) ist ca. 8-9%. Die maximale Steigerung der Transmission durch Kontakt und Druck wird mit einem Faktor von ca. 3,3 -3,4 angegeben. Dies würde bei Annahme von 9% und dem Faktor 3,4 eine Transmission von ca. 30,6 % ergeben. Die optische Leistung ist dann $\Phi e$ = 23,92 $\mu$W, was eine maximale Expositionszeit von ca. 59 Minuten ergibt. Da aber bei der maximalen Expositionsdauer von 59 Minuten ein durch Messung auf einer Fläche von $7,85 \times 10^{-3}$ cm$^2$ statt $7,07 \times 10^{-6}$ cm$^2$ bestimmter hoher Sicherheitsfaktor besteht, sind mögliche Schäden nicht zu erwarten.

Die thermische Belastung nach DIN EN ISO 15004-2:2014 erreicht durch die radiometrische Beurteilung, unter Berücksichtigung der maximalen Intensität durch Messung der Abstrahlcharakteristik, einen Wert von $E_{VIS-R}$ = 0,35 W/cm$^2$. Der Grenzwert von 0,7 W/cm$^2$ wird hier nicht erreicht.

Durch die direkte Wärmezufuhr, durch Abwärme der LED, ist ebenfalls keine Gefährdung zu erwarten. Die maximale Oberflächentemperatur der Kontaktfläche mit der Sklera, von 27,8 °C, ist weit entfernt von der maximal zulässigen Temperaturerhöhung von 10 - 20 °C im retinalen Pigmentepithel. So müsste eine Temperatur von 47 - 57 °C erreicht werden.

Die in Figur 1A schematisch dargestellte Ausführungsform erfüllt die Vorgaben der DIN EN ISO 15004-2:2014 und hat somit die Vorrausetzung als medizinisches Produkt zugelassen zu werden.

Beispiel 5: Eigenschaften des internen LED-Endoilluminators

Die in Figur 1B gezeigte Ausführungsform kombiniert Eigenschaften der in Figur 1A gezeigten Ausführungsform und der herkömmlichen Variante mit insertierter Lichtsonde. Sie ergibt eine ganzheitliche, sehr helle Ausleuchtung des intraokularen Raums. Dies ist die Folge der geweberhellenden Streuung und der direkten Beleuchtung durch die Lichtsonde. In Abbildung 1B ist eine sehr helle Ausleuchtung des Intraokularen Raums zu sehen, die durch den großen Abstand zur Retina (18 mm) und den geringen blauen Peak der LED eine geringere Gefährdung als vergleichbar helle Xenon-Illuminatoren, verursacht. Die Kontur des Lichtkegels der Lichtsonde verschwimmt durch die Erhellung des Gewebes und produziert eine sehr helle, ganzheitliche Ausleuchtung des intraokularen Raums.

Der Unterschied der Brechungsindizes des LED-Gehäuses (n = 1,5) und des Glaskörpers (n = 1,3367) verursacht, dass der Abstrahlwinkel im intraokularen Raum kleiner wird. An der Kontaktfläche (16) des LED-Gehäuses ist im Vergleich zu der in Figur 1A gezeigten Ausführungsform die Streuung größer und die Transmission kleiner, da kein mechanischer Druck auf die Sklera gegeben wird. Dies ergibt eine ganzheitliche Ausleuchtung, ohne einen lokalen Fokus, was eine Neupositionierung oder das Bewegen des Lichtkegels, unnötig macht.

Bei der Beurteilung der photochemischen Gefährdung wird die direkte Bestrahlung der Retina berechnet. Die mögliche Gefährdung der peripheren Retina, durch die transsklerale Beleuchtung ist in Tabelle 1 zu sehen. Hier werden die Werte mit 15% Transmission einbezogen da kein mechanischer Druck auf die Sklera erfolgt. Die reelle Tendenz liegt eher bei 10 %. Durch die direkte Bestrahlung ist eine Einsatzdauer von circa 6,5 Stunden möglich. Die transsklerale Beleuchtung lässt circa zwei Stunden zu. Folglich limitiert die transsklerale Beleuchtung die Einsatzdauer der in Figur 1B gezeigten Ausführungsform.

Tabelle 1: Radiometrische Beurteilung von LED 1 bei 3,6 V in 1 mm Abstand bei 15% Transmission

| **Photochemische Gefährdung** | | |
|---|---|---|
| Fläche | $7,85*10^{-3}$ | $[cm^2]$ |
| $\Phi_{e,rech.}$ | 11,8 | $[\mu W]$ |
| $\Phi_{e,rech.}$ | $1,18*10^{-5}$ | $[W]$ |
| $E_{e,rech.}$ | $1,5*10^{-3}$ | $[W/cm^2]$ |
| $E_{\lambda,rech.}$ | $8,53*10^{-5}$ | $[W/(cm^2*nm)]$ |
| $E_{A-R}$ | $1,40*10^{-3}$ | $[W/cm^2]$ |
| Grenzwert | 10 | $[J/cm^2]$ |
| | | |
| $t_{max}$ | 7164,61 | $[s]$ |
| | 119,41 | $[min]$ |
| | 1,99 | $[h]$ |

Die thermische Gefährdung nach DIN EN ISO 15004-2:2014 ist hier differenziert zu betrachten. An der Sklera direkt tritt eine thermische Belastung von $E_{VIS-R} = 0,35$ W/cm$^2$ auf. An der Retina, in 18 mm Abstand, ist eine thermische Belastung von $E_{VIS-R} = 10,1$ mW/cm$^2$ zu erwarten. Beide erreichen den Grenzwert von 0,7 W/cm$^2$ nicht.

Die direkte Wärmezufuhr durch Abwärme der LED ist um einiges höher als bei der in Figur 1A gezeigten Ausführungsform. Dies ist eine Folge des geringeren Abstandes zwischen LED-Chip und Sklera. Im Vergleich zu der in Figur 1A gezeigten Ausführungsform sind diese nur durch eine sehr dünne Epoxidharzschicht getrennt. Die maximal erreichte Temperatur auf dieser dünnen Epoxidharzschicht von 40,2 °C erreicht aber nicht den kritischen Bereich der Temperaturerhöhung im retinalen Pigmentepithel.

Die in Figur 1B schematisch dargestellte Ausführungsform erfüllt die Vorgaben der DIN EN ISO 15004-2:2014 und hat somit die Voraussetzung als medizinisches Produkt zugelassen zu werden.

**Patentansprüche**

1. Vorrichtung zur Beleuchtung des intraokularen Raumes (6) eines menschlichen oder tierischen Auges, welche

   a) eine Lichtquelle (2);
   b) ein Gehäuse (1), das die Lichtquelle umgibt;
   c) eine mit der Lichtquelle verbundene Spannungsquelle; und
   d) eine Fixiervorrichtung zum Fixieren des Gehäuses an der Sklera des zu beleuchtenden Auges
   aufweist, wobei die Fixiervorrichtung so ausgestaltet ist, dass das Gehäuse an einem Lidsperrer (12) anbringbar ist, so dass eine stabile Anbringung des Gehäuses auf der Sklera des menschlichen oder tierischen Auges durch Ausüben und Aufrechterhalten eines Anpressdrucks zwischen der Kontaktfläche des Gehäuses und der Sklera des menschlichen oder tierischen Auges ermöglicht ist; wobei die Lichtquelle eine thermische Belastung radiometrisch unter Berücksichtigung der maximalen Intensität der Abstrahlcharakteristik gemessen von weniger als 0,7 W/cm$^2$ erzeugt, und wobei die Lichtquelle den Augeninnenraum um nicht mehr als 10 °C erwärmt, und wobei das Spektrum der Lichtquelle keine detektierbare Strahlung von weniger als 400 nm Wellenlänge aufweist, wodurch die Phototoxizität der durch die Lichtquelle abgegebenen Strahlung auf der Retina des Auges gesenkt wird.

2. Vorrichtung zur Beleuchtung des intraokularen Raumes (6) eines menschlichen oder tierischen Auges, welche

   e) eine Lichtquelle (2);
   f) ein Gehäuse (1), das die Lichtquelle umgibt;
   g) eine mit der Lichtquelle verbundene Spannungsquelle; und
   h) eine Fixiervorrichtung zum Fixieren des Gehäuses an der Sklera des zu beleuchtenden Auges
   aufweist, wobei die Fixiervorrichtung so ausgestaltet ist, dass das Gehäuse eine konisch geformte Lichtsonde aufweist, die zur transkonjunktivalen Insertion geeignet ist, wobei sich der Durchmesser der Lichtsonde vom Gehäuse in Richtung von dem Gehäuse weg erhöht, und wobei der maximale Außendurchmesser am distalen Ende der Lichtsonde 20 Gauge ist; wobei die Lichtquelle eine thermische Belastung radiometrisch unter Berücksichtigung der maximalen Intensität der Abstrahlcharakteristik gemessen von weniger als 0,7 W/cm$^2$ erzeugt, und wobei die Lichtquelle den Augeninnenraum um nicht mehr als 10 °C erwärmt, und wobei das Spektrum der Lichtquelle keine detektierbare Strahlung von weniger als 400 nm Wellenlänge aufweist, wodurch die Phototoxizität der durch die Lichtquelle abgegebenen Strahlung auf der Retina des Auges gesenkt wird.

3. Vorrichtung nach Anspruch 1 zur transskleralen Beleuchtung des intraokularen Raumes eines menschlichen oder tierischen Auges, welche d) die Fixiervorrichtung zum Fixieren des Gehäuses an der äußeren Oberfläche der Sklera aufweist.

4. Vorrichtung gemäß einem der Ansprüche 1 bis 3, wobei das Gehäuse einen Diffusor aufweist, der eine zur Auflegung an die Sklera eines menschlichen oder tierischen Auges geeignete Kontaktfläche (16) aufweist, und der die in Richtung der Kontaktfläche hin emittierte Strahlung unregelmäßig streut.

5. Vorrichtung gemäß einem der Ansprüche 1, 3, oder 4, wobei das Gehäuse so an dem Lidsperrer anbringbar ist, dass die Position des Gehäuses relativ zur Position des Lidsperrers in Richtung zu der Sklera des menschlichen oder tierischen Auges hin oder von der Sklera weg einstellbar ist, wenn der Lidsperrer bestimmungsgemäß an dem Auge angebracht ist, wodurch der Anpressdruck zwischen der Kontaktfläche des Gehäuses und der Sklera einstellbar ist.

6. Vorrichtung gemäß Anspruch 5, wobei die Position des Gehäuses relativ zur Position des Lidsperrers in Richtung zu der Sklera des menschlichen oder tierischen Auges hin oder von der Sklera weg mittels Drehung des Gehäuses einstellbar ist, wenn der Lidsperrer bestimmungsgemäß an dem Auge angebracht ist, wobei das Gehäuse röhrenförmig ist, und axial in Richtung des zu beleuchtenden intraokularen Raumes ausgerichtet ist, und einen Gewindegang (11) auf der äußeren Oberfläche aufweist, wobei der Gewindegang über eine in die Fixiervorrichtung eingebrachte Aussparung mit der Fixiervorrichtung in Verbindung steht, wobei die Fixiervorrichtung mit dem Lidsperrer in einer fixen Position verbindbar ist.

7. Vorrichtung gemäß Anspruch 2, wobei die Fixiervorrichtung so ausgestaltet ist, dass das Gehäuse eine im Wesentlichen zylindrisch geformte Lichtsonde (3) aufweist, die zur transkonjunktivalen Insertion geeignet ist, wobei die Lichtsonde einen in Bezug auf das LED-Gehäuse proximalen und einen distalen Bereich aufweist, wobei der Durchmesser im proximalen Bereich im Wesentlichen konstant bleibt, und wobei sich der Durchmesser im distalen Bereich zunächst auf einen maximalen Außendurchmesser erhöht, und dann am distalen Ende der Lichtsonde vom Gehäuse in Richtung von dem Gehäuse weg wieder auf den Durchmesser des proximalen Bereiches verjüngt, und wobei der maximale Außendurchmesser der Lichtsonde 20 Gauge ist.

8. Vorrichtung gemäß einem der Ansprüche 1 bis 7, wobei die Vorrichtung eine Fixiervorrichtung gemäß einem der Ansprüche 1, 3, 5 oder 6, und eine Fixiervorrichtung gemäß Anspruch 2 oder 7 aufweist.

9. Vorrichtung gemäß einem der Ansprüche 1 bis 8, wobei die Lichtquelle eine Farbtemperatur von etwa 2.600 K bis etwa 3.500 K hat, wobei die CIE-Koordinaten von 0,35 - 0,45(x)/0,35 - 0,45(y) sind.

10. Vorrichtung gemäß einem der Ansprüche 1 bis 8, wobei die Lichtquelle eine einfarbige oder mehrfarbige LED ist, und/oder wobei die Lichtquelle eine organische Leuchtdiode (OLED) ist.

11. Vorrichtung gemäß einem der Ansprüche 1 bis 10, wobei das Gehäuse aus einem Polymer hergestellt ist, das Licht diffundiert, und/oder wobei das Gehäuse seitlich in Bezug auf die Linie, die den Strahlungsausgang der Lichtquelle mit dem zu beleuchtenden intraokularen Raum verbindet, abgeschirmt ist.

12. Verwendung der Vorrichtung nach einem der Ansprüche 1, 3 bis 6, oder 9 bis 11, wobei der Rückbezug der Ansprüche 9-11 auf Anspruch 2 ausgenommen ist, zur transskleralen nicht-invasiven Beleuchtung des intraokularen Raumes eines menschlichen oder tierischen Auges.

13. Verwendung nach Anspruch 12, wobei die Kontaktfläche des Gehäuses mit der Pars-Plana-Region der Sklera in Kontakt steht.

14. Verfahren zur nicht-invasiven transskleralen Beleuchtung des intraokularen Raumes eines menschlichen oder tierischen Auges, wobei das Verfahren die Verwendung gemäß Anspruch 12 umfasst, und wobei die Intensität der intraokularen Beleuchtung durch Erhöhung des Anpressdrucks zwischen der Kontaktfläche des Gehäuses und der Sklera gesteigert wird.

**Claims**

1. Device for illuminating the intraocular space (6) of a human or animal eye, comprising

   a) a light source (2);
   b) a housing (1) surrounding the light source; and
   c) a voltage source connected to the light source; and
   d) a fixing device for fixing the housing to the sclera of the eye to be illuminated,
   wherein the fixing device is configured, so that the housing is attachable to an eyelid speculum (12), so that a stable attachment of the housing on the sclera of the human or animal eye is possible by exerting and maintaining a contact pressure between the contact surface of the housing and the sclera of the human or animal eye; wherein the light source produces a thermal load radiometrically measured with respect to the maximum intensity of the radiation characteristic of less than 0.7 W/cm$^2$, and wherein the light source heats the inner space of the eye by no more than 10°C, and wherein the spectrum of the light source comprises no detectable radiation of less than 400 nm wavelength, thereby reducing the phototoxicity of the radiation emitted by the light source on the retina of the eye.

2. Device for illuminating the intraocular space (6) of a human or animal eye, comprising

   e) a light source (2);
   f) a housing (1) surrounding the light source; and
   g) a voltage source connected to the light source; and
   h) a fixing device for fixing the housing to the sclera of the eye to be illuminated,
   wherein the fixing device is configured, so that the housing comprises a conically shaped light probe, which is suitable for transconjunctival insertion, wherein the diameter of the light probe increases from the housing in a direction away from the housing, and wherein the maximum outer diameter at the distal end of the light probe is 20 gauge;

wherein the light source produces a thermal load radiometrically measured with respect to the maximum intensity of the radiation characteristic of less than 0.7 W/cm$^2$, and wherein the light source heats the inner space of the eye by no more than 10°C, and wherein the spectrum of the light source has no detectable radiation of less than 400 nm wavelength, thereby reducing the phototoxicity of the radiation emitted by the light source on the retina of the eye.

3. Device according to claim 1 for transscleral illumination of the intraocular space of a human or animal eye, comprising (d) the fixing device for fixing the housing to the outer surface of the sclera.

4. Device according to any one of claims 1 to 3, wherein the housing comprises a diffuser, which comprises a contact surface (16) suitable for placement at the sclera of a human or animal eye, and which irregularly scatters the radiation emitted towards the contact surface.

5. Device according to any one of claims 1, 3, or 4, wherein the housing is attachable to the eyelid speculum, so that the position of the housing relative to the position of the eyelid speculum is adjustable in a direction toward or away from the sclera of the human or animal eye, when the eyelid speculum is attached to the eye, whereby the contact pressure between the contact surface of the housing and the sclera is adjustable.

6. Device according to claim 5, wherein the position of the housing relative to the position of the eyelid speculum is adjustable in a direction toward or away from the sclera of the human or animal eye by rotation of the housing, when the eyelid speculum is properly attached to the eye, wherein the housing is Tube-shaped and axially oriented in a direction toward the intraocular space to be illuminated and comprises a threaded passage (11) on the outer surface, wherein the threaded passage communicates with the fixing device via a recess formed in the fixing device, wherein the fixing device is connectable to the eyelid speculum in a fixed position.

7. Device according to claim 2, wherein the fixing device is configured, so that the housing comprises a substantially cylindrically shaped light probe (3) suitable for transconjunctival insertion, wherein the light probe comprises a proximal region and a distal region with respect to the LED housing, wherein the diameter in the proximal region remains substantially constant, and wherein the diameter in the distal region initially increases to a maximum outer diameter, and then tapers back to the diameter of the proximal region at the distal end of the light probe from the housing in a direction away from the housing, and wherein the maximum outer diameter of the light probe is 20 gauge.

8. Device according to any one of claims 1 to 7, wherein the device comprises a fixing device according to any one of claims 1, 3, 5 or 6, and a fixing device according to claim 2 or 7.

9. Device according to any one of claims 1 to 8, wherein the light source comprises a color temperature of about 2,600 K to about 3,500 K, wherein the CIE coordinates are from 0.35 - 0.45(x)/0.35 - 0.45(y).

10. Device according to any one of claims 1 to 8, wherein the light source is a single-color or multicolor LED, and/or wherein the light source is an organic light emitting diode (OLED).

11. Device according to any one of claims 1 to 10, wherein the housing is made of a polymer that diffuses light, and/or wherein the housing is shielded laterally with respect to the line connecting the radiation output of the light source to the intraocular space to be illuminated.

12. Use of the device of any one of claims 1, 3 to 6, or 9 to 11, wherein the dependency of claims 9-11 to claim 2 is excluded, for transscleral non-invasive illumination of the intraocular space of a human or animal eye.

13. Use according to claim 12, wherein the contact surface of the housing is in contact with the pars plana region of the sclera.

14. Method of non-invasive transscleral illumination of the intraocular space of a human or animal eye, wherein the method comprises the use according to claim 12, and wherein the intensity of the intraocular illumination is increased by increasing the contact pressure between the contact surface of the housing and the sclera.

**Revendications**

1. Dispositif conçu pour éclairer l'espace intraoculaire (6) d'un œil humain ou animal, comprenant

   a) une source de lumière (2) ;
   b) un boîtier (1) entourant ladite source de lumière ;
   c) une source de tension connectée à ladite source de lumière ; et
   d) un dispositif de blocage à demeure, affecté au blocage à demeure dudit boîtier sur la sclère de l'œil à éclairer, ledit dispositif de blocage à demeure étant agencé de façon telle que le boîtier puisse être mis en place sur un spéculum ophtalmologique (12) en vue de permettre une mise en place stable dudit boîtier, sur la sclère de l'œil humain ou animal, en exerçant et en entretenant une pression de contact entre la surface de contact dudit boîtier et la sclère dudit œil humain ou animal ;
   sachant que la source de lumière engendre une charge thermique inférieure à 0,7 W/cm$^2$, mesurée par radiométrie avec prise en compte de l'intensité maximale de la caractéristique de rayonnement, et sachant que ladite source de lumière ne réchauffe pas l'espace interne de l'œil de plus de 10 °C, le spectre de ladite source de lumière ne présentant aucun rayonnement détectable inférieur à 400 nm de longueur d'onde, la phototoxicité du rayonnement émis par ladite source de lumière, sur la rétine de l'œil, s'en trouvant ainsi réduite.

2. Dispositif conçu pour éclairer l'espace intraoculaire (6) d'un œil humain ou animal, comprenant

   e) une source de lumière (2) ;
   f) un boîtier (1) entourant ladite source de lumière ;
   g) une source de tension connectée à ladite source de lumière ; et
   h) un dispositif de blocage à demeure, affecté au blocage à demeure dudit boîtier sur la sclère de l'œil à éclairer, ledit dispositif de blocage à demeure étant agencé de façon telle que le boîtier comporte une sonde lumineuse de forme conique se prêtant à l'insertion transconjonctivale, sachant que le diamètre de la sonde lumineuse croît à partir du boîtier, dans une direction s'éloignant dudit boîtier, et sachant que le diamètre extérieur maximal est de calibre 20 à l'extrémité distale de ladite sonde lumineuse ;
   sachant que la source de lumière engendre une charge thermique inférieure à 0,7 W/cm$^2$, mesurée par radiométrie avec prise en compte de l'intensité maximale de la caractéristique de rayonnement, et sachant que ladite source de lumière ne réchauffe pas l'espace interne de l'œil de plus de 10 °C, le spectre de ladite source de lumière ne présentant aucun rayonnement détectable inférieur à 400 nm de longueur d'onde, la phototoxicité du rayonnement émis par ladite source de lumière, sur la rétine de l'œil, s'en trouvant ainsi réduite.

3. Dispositif selon la revendication 1, dévolu à l'éclairage transscléral de l'espace intraoculaire d'un œil humain ou animal, comportant
   d) le dispositif de blocage à demeure affecté au blocage à demeure du boîtier au niveau de la surface extérieure de la sclère.

4. Dispositif selon l'une des revendications 1 à 3, le boîtier étant pourvu d'un diffuseur qui est muni d'une surface de contact (16) se prêtant à l'application sur la sclère d'un œil humain ou animal et qui diffuse, de façon irrégulière, le rayonnement émis en direction de ladite surface de contact.

5. Dispositif selon l'une des revendications 1, 3 ou 4, le boîtier pouvant être mis en place sur le spéculum ophtalmologique de façon telle que l'emplacement dudit boîtier, par rapport à l'emplacement dudit spéculum ophtalmologique, puisse être réglé en direction de la sclère de l'œil humain ou animal, ou avec éloignement vis-à-vis de ladite sclère, lorsque ledit spéculum ophtalmologique est mis en place sur l'œil en conformité avec sa destination, permettant ainsi un réglage de la pression de contact entre la surface de contact dudit boîtier et ladite sclère.

6. Dispositif selon la revendication 5, l'emplacement du boîtier par rapport à l'emplacement du spéculum ophtalmologique pouvant être réglé en direction de la sclère de l'œil humain ou animal, ou avec éloignement vis-à-vis de ladite sclère, par rotation imprimée audit boîtier lorsque ledit spéculum ophtalmologique est mis en place sur l'œil en conformité avec sa destination, sachant que ledit boîtier est de forme tubulaire, est orienté axialement en direction de l'espace intraoculaire devant être éclairé, et est doté d'un filetage (11) sur la surface extérieure, ledit filetage étant en liaison avec le dispositif de blocage à demeure par l'intermédiaire d'un évidement pratiqué dans ledit dispositif de blocage à demeure, lequel dispositif de blocage à demeure peut être relié audit spéculum ophtalmologique en un emplacement fixe.

**7.** Dispositif selon la revendication 2, le dispositif de blocage à demeure étant agencé de façon telle que le boîtier comporte une sonde lumineuse (3) de forme substantiellement cylindrique se prêtant à l'insertion transconjonctivale, laquelle sonde lumineuse comprend une région proximale par rapport audit boîtier à DEL, et une région distale, sachant que le diamètre demeure substantiellement constant dans la région proximale et que ledit diamètre croît tout d'abord jusqu'à un diamètre extérieur maximal, dans la région distale, et s'amenuise ensuite de nouveau pour reprendre le diamètre de ladite région proximale à l'extrémité distale de ladite sonde lumineuse à partir du boîtier, dans une direction s'éloignant dudit boîtier, ledit diamètre extérieur maximal de la sonde lumineuse étant de calibre 20.

**8.** Dispositif selon l'une des revendications 1 à 7, ledit dispositif étant nanti d'un dispositif de blocage à demeure conforme à l'une des revendications 1, 3, 5 ou 6, et d'un dispositif de blocage à demeure conforme à la revendication 2 ou 7.

**9.** Dispositif selon l'une des revendications 1 à 8, la source de lumière présentant une température de coloration d'environ 2 600 K à environ 3 500 K, les coordonnées CIE étant de 0,35 - 0,45(x)/ 0,35 - 0,45(y).

**10.** Dispositif selon l'une des revendications 1 à 8, la source de lumière étant une DEL monochrome ou polychrome, et/ou ladite source de lumière étant une diode électroluminescente organique (DELO).

**11.** Dispositif selon l'une des revendications 1 à 10, le boîtier étant fabriqué en un polymère qui diffuse de la lumière, et/ou ledit boîtier étant blindé latéralement par rapport à la ligne qui relie la sortie du rayonnement de la source de lumière à l'espace intraoculaire devant être éclairé.

**12.** Utilisation du dispositif conforme à l'une des revendications 1, 3 à 6 ou 9 à 11, excluant le rattachement des revendications 9-11 à la revendication 2, pour l'éclairage transscléral non invasif de l'espace intraoculaire d'un œil humain ou animal.

**13.** Utilisation selon la revendication 12, la surface de contact du boîtier étant en contact avec la zone *pars plana* de la sclère.

**14.** Procédé d'éclairage transscléral non invasif de l'espace intraoculaire d'un œil humain ou animal, ledit procédé incluant l'utilisation conforme à la revendication 12, et l'intensité de l'éclairage intraoculaire étant augmentée par accroissement de la pression de contact entre la surface de contact du boîtier et la sclère.

Fig. 1A

Fig. 1B

Figur 2

Figur 3

## Figur 4

A

B

Figur 5

## Figur 6

A

B

Figur 7

A

500µm

B

500µm

# Figur 8

**IN DER BESCHREIBUNG AUFGEFÜHRTE DOKUMENTE**

*Diese Liste der vom Anmelder aufgeführten Dokumente wurde ausschließlich zur Information des Lesers aufgenommen und ist nicht Bestandteil des europäischen Patentdokumentes. Sie wurde mit größter Sorgfalt zusammengestellt; das EPA übernimmt jedoch keinerlei Haftung für etwaige Fehler oder Auslassungen.*

**In der Beschreibung aufgeführte Patentdokumente**

- US 20040004846 A1

- DE 102010016629 A1